(19) **Europäisches Patentamt / European Patent Office / Office européen des brevets**

(11) **EP 0 958 046 B1**

(12) **EUROPEAN PATENT SPECIFICATION**

(45) Date of publication and mention
of the grant of the patent:
**26.10.2011 Bulletin 2011/43**

(21) Application number: **96944310.0**

(22) Date of filing: **11.12.1996**

(51) Int Cl.:
*B01J 20/32* (2006.01)     *G01N 33/49* (2006.01)
*B01L 3/14* (2006.01)

(86) International application number:
**PCT/US1996/019713**

(87) International publication number:
**WO 1997/021488 (19.06.1997 Gazette 1997/26)**

(54) **CELL SEPARATION COMPOSITION, KIT AND METHOD**

ZUSAMMENSETZUNG, KIT UND VERFAHREN ZUR ZELLTRENNUNG

COMPOSITION POUR SEPARATION DE CELLULES, TROUSSE ET PROCEDE ASSOCIES

(84) Designated Contracting States:
**AT BE CH DE DK ES FI FR GB GR IE IT LI LU MC NL PT SE**

(30) Priority: **11.12.1995 US 570120**
         **11.12.1995 US 570397**

(43) Date of publication of application:
**24.11.1999 Bulletin 1999/47**

(73) Proprietor: **DENDREON CORPORATION**
**Seattle, WA 98121 (US)**

(72) Inventors:
  • **VAN VLASSELAER, Peter**
    **Sunnyvale, CA 94087 (US)**
  • **PALATHUMPAT, Varghese**
    **Fremont, CA 94539 (US)**

(74) Representative: **Vossius & Partner**
**Siebertstrasse 4**
**81675 München (DE)**

(56) References cited:
**EP-A- 0 574 227     WO-A-96/07097**
**DE-A- 3 222 452     US-A- 4 927 750**

• **JOURNAL OF APPLIED POLYMER SCIENCE, vol. 37, no. 10, 20 May 1989, NEW-YORK, pages 2921-2931, XP000083825 MARK CHAIMBERG: "GRAFT POLYMERIZATION OF PVP ONTO SILICA"**
• **SCANDINAVIAN JOURNAL OF HAEMATOLOGY, vol. 24, 1980, COPENHAGEN, pages 254-262, XP000670657 OLOFSSON: "SEPARATION OF HUMAN BONE MARROW CELLS IN DENSITY GRADIENTS OF POLYVINYLPYRROLIDONE COATED SILICA GEL"**

## Description

### Field of the Invention

The present invention relates to compositions, kits and methods for cell separation.

### Background of the Invention

Separation of cells and cellular components, long an important tool in basic research and diagnostic applications, is becoming increasingly important in the clinical setting. The use of cell transplantation methods, such as bone marrow transplantation, in human therapeutics has necessitated cell separation procedures that are not only rapid and reproducible but which also produce viable, safe, non-toxic cell composition. It is also desirable that such isolation procedures are suitable for isolating cells that are relatively low in abundance. For example, following chemotherapy for certain types of cancer, many patients now receive "stem cell transplants" which consist of an enriched fraction of hematopoietic progenitor cells isolated from various tissues, such as bone marrow, peripheral blood or cord blood. Such progenitor cells constitute only about 1% of the total number of cells present in these tissues.

Density gradient centrifugation is a popular technique for separating and isolating cells and cellular components. This method exploits the phenomenon that cells partition in a defined density medium according to their buoyant densities. Such a medium may be a solution or a suspension of microparticles. Examples of media commonly used in density gradient separation include sucrose, dextran, bovine serum albumin (BSA), FICOLL diatrizoate (Pharmacia), FICOLL metrizoate (Nycomed), PERCOLL® (Pharmacia), metrizamide, and heavy salts such as cesium chloride. Exposure of cells to many of these media produces impaired biological function of the cells and/or contamination of the preparation by toxic components. For example, BSA and FICOLL are known to cause unwanted cell aggregation at physiological pH. In addition, these media are not generally amenable to sterilization by autoclave or irradiation in "final form" - that is, in a concentration, ionic solution and container that is ready to use in isolating a particular cell type.

One cell separation medium that has been widely used in preparation of blood cell fractions is PERCOLL® (a registered trademark of Pharmacia Fine Chemicals). PERCOLL® is a colloidal silica particle treated by a curing process to form a polyvinylpyrrolidone coating on each particle. While PERCOLL® is fairly stable at physiological pH, there are some limitations to its general usefulness in isolating cells for therapeutic purposes. For example, the medium is difficult to sterilize, since it is not stable to autoclaving or ionizing irradiation after it is diluted in a physiological salt solution. These properties limit the usefulness of the product for clinical applications involving re-introduction of separated cells into humans or anywhere else that finally sterilized material is required.

U.S. Patent 4,927,749 provides an organosilanized colloidal silica (OCS) particle (OCSP) preparation for density gradient separation that overcomes some of the problems discussed above. While it is stable to sterilization by heat and ionizing irradiation when diluted in a physiological salt solution, when sterilized by ionizing radiation, OCS particle preparations are toxic to certain rare cell types, such as dendritic cells, natural killer cells, natural suppressor cells, cytotoxic T cells, and in particular, hematopoietic progenitor (CD34+) cells. Since final sterilization by ionizing radiation may be preferred for certain applications, particularly those involving containment of the material in plastic consumable vessels, the use of this material for clinical applications may be limited. Moreover, it has been found that the material induces clumping or aggregation of cells, resulting in significantly reduced yields of such rare cells in functional form.

The present invention provides compositions and methods that are particularly suitable for isolation of "rare" cell fractions--*e.g.*, cell types that constitute less than about 1 % of total cells in a cell population. In particular, the invention includes a colloidal silica cell separation media that improves the yield and functional potential of cells by reducing cell aggregation and cell toxicity. It is the discovery of the present invention that inclusion of a polylactam, such as the gamma polylactam polyvinylpyrrolidone (PVP), in the solution in which colloidal silica density gradient material is suspended markedly reduces aggregation of rare blood cells, resulting in improved yields. It is a further discovery of the present invention that addition of the polylactam to the medium prevents loss of clonogenic potential during density gradient isolation of certain hematopoietic progenitor cells by reducing the irradiation-induced toxicity of OCS solutions.

The invention also provides methods for isolation of specific cell types that are important in diagnostic and therapeutic methods--including, for example, hematopoietic progenitor (CD34+) cells isolated from bone marrow, selected tumor cells, dendritic cells, natural killer (NK) cells, cytotoxic T-lymphocytes (CTL) and natural suppressor cells. The invention also provides for the depletion of cell types that may interfere with a particular clinical outcome, such as CTL.

### Summary of the Invention

In one aspect, the invention includes compositions for use in cell separation consisting of a silanized colloidal silica particle-based cell separation medium containing at least about 0.05% polylactam, such as polyvinylpyrrolidone (PVP), present at a concentration between about 0.1 and about 10 percent. In a preferred embodiment, the separation medium is composed of a suspension of organosilanized colloidal silica particles. An advantage of this composition is that it can be sterilized, as by autoclaving or by ionizing radiation, without imparting toxic effects on the cells subsequently separated herein.

While particles comprising the media of the invention can range from about 0.003 to 50 microns, colloidal silica particles are usually on the average of 10-50 nm in diameter.

Compositions of the invention are particularly suited for use in isolating a selected rare blood cell from a cell mixture. According to methods described herein, in such cases, the medium is prepared such that the particle suspension has a specific density within about 0.0005 gr/ml of said selected cell. Exemplary rare cell types include hematopoietic progenitor $CD34^+$ cells, dendritic cells, natural killer cells, natural suppressor cells, cytotoxic T cells, tumor cells and nucleated fetal cells.

Hematopoietic progenitor $CD34^+$ cells may be isolated from peripheral blood mononuclear cells, in which case the preferred organosilanized colloidal silica particle suspension has a specific density of 1.0605 gr/ml. When hematopoietic progenitor $CD34^+$ cells are isolated from bone marrow cells, the organosilanized colloidal silica particle suspension preferably has a specific density of 1.0685 gr/ml.

Dendritic cells are also isolated using- compositions described above. In this case, suspensions having densities of 1.0770 gr/ml, 1.0720 gr/ml, 1.0650 gr/ml, 1.0610 gr/ml, and 1.0565 gr/ml may be utilized.

The invention also includes a kit for cell separation. The kit consists of a centrifugable container, and a silanized colloidal silica particle-based cell separation suspension as described above.

Also included in the present invention is a method of sterilizing a silanized silica particle-based cell separation medium, where the separation medium is subjected to autoclaving or ionizing radiation in the presence of at least about 0.05 percent, and preferably between about 0.1 and 10 percent, polylactam.

More generally, the invention includes a method of isolating a selected rare blood cell from a cell mixture where the mixture is first layered onto a silanized colloidal silica particle-based cell separation medium having a specific density within about 0.0005 gr/ml of the selected cell. The mixture is then centrifuged. According to a preferred embodiment, the centrifugation is carried out in a so-called "cell-trap" tube which contains: (i) side walls and a closed bottom, and (ii) a constriction member disposed within the tube, where the constriction member is positioned and constructed to retain fluid in the bottom portion of the tube below the constriction member, when the tube is inverted. Also, in this configuration, prior to centrifugation, the tube is filled with the separation medium to a level extending above said constriction member to a level above an opening formed by said constriction member, such that cells which are captured at an interface between the cell-separation medium and a lower-density medium, after centrifugation, are discharged with the lower-density medium when the tube is inverted. The tube may also include a closed top, a first port in top through which fluid material can be introduced into the tube, a second port in the top, and a closed fluid channel communicating the second port with the bottom of the tube below the constriction member.

This configuration can be used to isolated functional hematopoietic progenitor cells at separation medium concentrations of 1.0605 gr/ml (from peripheral blood cell mixtures) and 1.0685 gr/ml (from bone marrow). Tumor cells are also isolated by this method using a cell separation medium of a specific density selected from the range 1.0490-1.0580 gr/ml. Likewise, dendritic cells can be isolated using separation media having specific densities of 1.0770 gr/ml, 1.0720 gr/ml, 1.0650 gr/ml, 1.0610 gr/ml, and 1.0565 gr/ml.

Cytotoxic T lymphocytes may be isolated using a specific density of 1.0720 gr/ml, 1.0610 gr/ml or 1.0565 gr/ml.

The forgoing separation methods may be enhanced by adding a suspension which includes a silanized silica particle to which is attached a cell antigen-specific binding molecule. Such addition may be used to deplete unwanted cell types from a preparation, such as T-cell lymphocytes.

## Brief Description of the Figures

FIG. 1 shows recovery of functional hematopoietic progenitor cells (colony forming units, CFU) after incubation with gamma-irradiated 0.5% PVP in PBS, gamma-irradiated OCS particle-based density gradient material (OCSP), or gamma-irradiated OCS particle-based density gradient material supplemented with 0.5% PVP (OCSP+0.5% PVP);

FIG. 2 shows the effect of various concentrations of PVP present during sterilization of OCS particles (OCSP) on gamma-irradiation-induced restriction of hematopoietic progenitor cell function (CFU);

FIG. 3 shows the distribution of peripheral blood progenitor cells (PBPC) in interface and pellet fractions of density gradients formed from colloidal silica particles in the absence (OCSP) and presence of 0.5% PVP (OCSP+PVP);

FIG. 4 shows the distribution of hematopoietic progenitor cells ($CD34^+$) cells in interface and pellet fractions of density gradients formed from colloidal silica particles (OCSP) in the absence and presence of 0.5% PVP (OCSP+PVP);

FIG. 5 shows a comparison of non-specific depletion of peripheral blood mononucleas cells (PBMC) associated with density adjusted cell sorting (DACS) beads coated with two types of GAM-IgG preparations;

FIG. 6 shows non-specific loss of CD34+ cells with DACS coated with intact boat Anti-Mouse (GAM)-IgG or F(ab)2 GAM-IgG;

FIG. 7 shows non-specific loss of CD34+ cells with DACS beads coated with intact GAM-IgG in either a random or a site-specific orientation;

FIG. 8 shows percent recovery of total cells, CD34+ cells, CTL's and red blood cells (RBC) from the combined interface + pellet after centrifugation of bone marrow cells on an OCS gradient;

FIG. 9 shows the percent recovery of total cells, CD34+ cells, CTL's and red blood cells (RBC) from the interface of the gradient following centrifugation;

FIG. 10 shows distribution of natural suppressor cell activity in different density fractions;

FIG. 11 shows distribution of natural killer cells in different density fractions;

FIG. 12 shows results of experiments in which peripheral blood buffy coat samples were depleted of human B lymphoma cells by DACS;

FIG. 13 shows recovery of CD34+ cells in the depleted peripheral blood buffy coat samples of FIG. 12;

FIG. 14 shows a sterilized centrifuge kit assembled in accordance with the invention; and

FIG. 15 shows a closed system centrifugation device suitable for inclusion in a kit of the invention.

## Detailed Description of the Invention

The invention encompasses cell density separation media suitable for separation of cells, and particularly for separation or isolation of cells to be transplanted into humans. The invention also includes methods for isolating specific cell types, preferably using the new medium composition.

As described in the sections that follow, the media of the invention have been developed in view of the applicants' discovery that cell toxicity is significantly reduced when a solution of a gamma polylactam such as polyvinylpyrrolidone is added to a suspension of organosilanized colloidal silica (OCS) particles used in density gradient separation of the cells.

In particular, inclusion of the polylactam prevents loss of clonogenic potential in rare populations of hematopoietic progenitor cells that occurs when such cells are suspended in a suspension of sterile silanized silica particles. In addition, the present invention includes the discovery that inclusion of polylactams such as polyvinylpyrrolidone in the density gradient medium also reduces aggregation and loss of clonogenic potential of hematopoietic progenitor cells that occurs without ionizing radiation.

### I. Definitions

"Colloidal silica" refers to an aqueous suspension of colloidal particles formed by polymerization of monosilicic acid from $SiO_2$ dissolved in water.

"Silanized silica particles" refers to a particulate silica composition to which is covalently linked an silane coating. "Organosilanized colloidal silica (OCS) particles" refers to a colloidal silica composition to which is covalently attached an organosilane coating. U.S. Patent 4,927,749 describes how to prepare such a composition.

"Rare cell populations" refers to cells that constitute less than about 1 % of the total white blood cell (WBC) count in the blood of a healthy individual. Examples of rare blood cells include CD34+ hematopoietic progenitor cells, which constitute about 1 % of while blood cells (WBC's), natural killer cells, Natural suppressor cells, dendritic cells, and cytotoxic T cells. Also included in this definition are cells from exogenous sources, including circulatory fetal cells in maternal blood.

"Cell toxicity", "toxic to cells", and similar phrases herein refer to any diminution in cell viability or biological function that is measurable in a cell population. With reference to the hematopoietic progenitor cells described herein, the term most commonly refers to diminution in clonogenic potential, as evidenced by reduced yield of cells capable of forming hematopoietic colonies (CFU).

A "polylactam" is a polymer containing pendant lactam (cyclic amide) groups attached to the polymer backbone. The polymer may be a homopolymer or a co-polymer. The pendant lactam groups will generally have ring sizes containing from 3-6 carbon atoms. One preferred type of polylactam for use in the present invention is a polyvinyl lactam, such as polyvinylpyrrolidone, which has a 4 carbon ring.

"Dendritic cells", or "DC" are immune cells that are negative for expression of CD3, CD4, CD8, CD14, CD16 and CD20, and positive for expression of HLA-DR (*i.e.*, class II MHC). Dendritic cells exhibit a distinctive morphology-that is, they are large veiled cells which extend dendrites when cultured *in vitro.*

A "tumor cell antigen" is a molecule, generally a protein, that is exposed on the surface of a particular tumor cell. Examples of tumor cell antigens include CD9, CD10, CD19 and CD20 present on B cell lymphoma cells; tumor cell antigens that are specific for certain breast tumors include Her2/Neu and estrogen receptors.

In the context of the present invention, the term "isotonic" means having an osmolality that is within the range tolerated by the cell, usually about 280-340 mOsm/kg $H_2O$, and preferably, for human cells, about 280 mOsm/kg $H_2O$, depending on the cell type and source.

II. Cell Separation Medium

This section describes various cell separation media that can be used to isolate cells from cell mixture. Preferred methods of cell separation described in the sections which follow include use of a cell separation material, such as a density gradient material, having a specific gravity between 1.0000 gr/ml and 2.0000 gr/ml, preferably between 1.0300 gr/ml and 1.2000 gr/ml, that is accurate within $\pm$ 0.0005 gr/ml, preferably $\pm$ 0.0002 gr/ml of the specific gravity of the desired cell.

A. Density Gradient Media

Preferred density gradient media are colloidal silica suspensions having particles ranging from 0.002 to 50 microns. One useful medium is PERCOLL®, a colloidal silica particle suspension which is not silanized and which is available from Pharmacia Fine Chemicals (Piscataway, N.J.). More-preferred is an organosilanized colloidal silica particle suspension as described in U.S. Patent 4,927,749, and treated with PVP, as described below.
According to an important feature of the invention, the density gradient solution should be prepared and adjusted to a pre-determined density. In addition, the osmolality should be adjusted in the range of 280 to 320 mOsmlkg $H_2O$ to preserve cell integrity, and the pH should preferably be in the range from 6.8 to 7.8, and most preferably pH 7.4, for maintaining a physiologically isotonic density gradient, prior to use. The osmolality and pH may vary depending upon the particular conditions under which the density gradient separation method is performed. For example, the temperature at which the samples are maintained or centrifuged may necessitate modifications to the osmolality and/or pH of the density gradient material, in order to maintain the appropriate density. Such modifications of the osmolality and pH will be apparent to those skilled in the art.
A preferred density gradient material for use in separating cells in accordance with the present invention is an organosilanized colloidal silica (OCS) particle suspension, such as are disclosed in U.S. Patent 4,927,749 to Dorn.
In a preferred embodiment, the OCS density gradient material is diluted to an appropriate specific density in a physiological salt solution supplemented with polyvinylpyrrolidone (PVP) such as PVP-10 available from Sigma Chemical Co. (St. Louis, MO). According to an important feature, as discussed below, it has been found that inclusion of at least 0.5% PVP improves the yield of functional rare blood cell types. In addition, such a concentration of PVP facilitates sterilization of the OCS density gradient material by ionizing radiation, such as E-beam or gamma irradiation. In the absence of PVP, cells exposed to the medium experience loss of functional capacity-in the case of CD34[+] cells, evidenced by loss of ability to form colonies (reduction of CFU). Generally, the amount of PVP to be added to an OCS cell separation suspension will depend on the subsequent purpose and treatment of the preparation. For preserving functional activity of cells exposed to the OCS preparation for relatively short periods of time, a concentration of 0.5% (wt/wt) PVP generally suffices. For longer exposures and exposure to ionizing radiation, higher concentrations of PVP may be required-as high as about 3-8% (wt/wt).

B. Silanized Silica Particles

The methods and compositions described herein are applicable to a number of uses of silanized silica particles in conjunction with cell separation. The particles can be silanized by any of a number of methods known in the art. U.S. Patent 4,927,749, describes preparation of OCS particles that are particularly useful in preparing the compositions used in the present invention. In one embodiment, OCS particles ranging in size from 3-22 nm, and particularly from 13-18 nm, form a stable suspension that acts as a density gradient medium for separating heterogeneous mixtures of cells on the basis of buoyant density.
In addition to forming density gradient materials, silanized silica particles can also be used in other aspects of cell separation. For example, particles used in certain positive or negative selection steps may be treated in accordance with the methods described herein. Such particles are typically silanized silica microspheres or beads having diameters ranging up to about 50 $\mu$m (microns), and conventionally, within the range of about 1-5 $\mu$m (microns). Such particles are silanized according to methods well known in the art, for example, using 3-aminopropyltriethoxysilane, (3-iodopropyl) trimethoxysilane, [1-9trimethoxysilyl)-2(m-(or p) chloromethyl)-phenyl] ethane, or 3-glycidyl-oxypropyltrimethoxysilane (GPMS). Typically, these particles have as covalent attachments specific antigen binding molecules, such as antibodies, lectins or other cell binding agents. When added to a cell mixture, the microspheres bind to cells having a specific target antigen or antigens, and modify (increase or decrease) the density of the cells.
Polyvinylpyrrolidone (PVP) has been added to suspensions of non-silanized colloidal silica particles (Pertoft, et al., Exp. Cell Res. 46: 621-623 (1966); Pertoft, et al., Exp. Cell Res. 50: 355-368 (1968); Wolff, et al., J. Cell Biol., 55:

579-585 (1972); Pertoft, et al., Exp. Cell Res., 110: 449-457 (1977)). However, free PVP in solution has been reported to have undesirable effects on some cell types (Pertoft, et al., Exp. Cell Res. 50: 355-368 (1968)). This lead to development of a thermal curing process that produces a PVP coating adsorbed to the colloidal silica particle, and which is substantially free of free PVP. (Pertoft, et al., Anal. Biochem. 88: 271-282 (1978)). This composition is commercially available as PERCOLL®.

However, PERCOLL cannot be sterilized by conventional means (autoclaving or ionizing radiation) when diluted in a physiological salt solution at a final concentration suitable for cell separation. This limits its usefulness in applications that require a finally sterilized density gradient material in a physiological medium.

As noted above, a colloidal silica particle-based preparation having a covalent organosilane coating has been found to have utility in separating various blood cells (U.S.

Patent 4,927,749). The organosilane coating reduces cell toxicity and eliminates aggregation of the colloidal silica particles themselves in the presence of physiological salt and protein. However organosilane-coated colloidal silica (OCS) particles have certain disadvantages when used to separate or purify specific rare blood cells from blood and other sources. Use of such particles may result in reduced recovery of such rare cells. This is exemplified below in conjunction with isolation of an important hematopoietic progenitor cell characterized antigenically as a CD34[+] cell, which can be isolated from blood or bone marrow. Specifically, these rare blood progenitor cells aggregate or otherwise increase in density, when suspended in OCS particle-based density material. Moreover, sterilization of an OCS particle-based suspension by ionizing radiation results in a composition that is toxic to these cells. Experiments demonstrating these results and the improvements provided by the discoveries of the present invention are presented in the sections that follow.

C. Protection by Polylactams against Radiation-induced Toxicity of Silanized Silica Particles

There are a number of methods of sterilizing density gradient materials. Silica-particle based density gradient materials can be sterilized by autoclaving or by filtration, according to methods known in the art. One method that is particularly useful is sterilization by ionizing radiation. This method may be preferred when the prepared density material is to be dispensed in plastic consumable containers, such as in pre-filled centrifuge tubes, bags, or syringes and the like for clinical separations, or when it is to be dispensed in any heat-sensitive containers, such as plastic bottles. Unlike other methods of sterilization, irradiation-based sterilization methods can be carried out on the end-product container as a one-step process. That is, when it is necessary to sterilize both a container and a liquid or suspension in the container, there is no need to separately sterilize the container itself, as is required when filtration methods are used, and there is no need to transfer the material from a sterilizing vat to individual containers, as would normally occur with heat-sterilization (autoclave).

Techniques for sterilizing medical devices by ionizing radiation are known in the art. Commonly used methods applicable to the present invention include gamma irradiation and electron beam (E-beam) radiation. While each of these methods involves a different type of energy source, both methods involve passing the device through a radiation field to achieve a specific radiation dose to the device. Typically, sterilization of a medical device requires a dose of between 10 and 30 kiloGrays (kGy). However, as described below, when either of these methods is used to sterilize organosilanized colloidal silica, the material becomes toxic to hematopoietic cells, unless a polylactam such as PVP is present in the particle suspension during irradiation.

The polylactams used in the present invention are preferably soluble polymers such as the KOLLIDON® series of polyvinylpyrrolidones (BASF, Ludwigshafen, Germany). These polymers are generally available in polymer molecular, weights ranging from 2000 to 350,000, and are rated by their intrinsic viscosities or "K values." In experiments carried out in support of the present invention, preparations having average molecular weights of about 10,000 were used.

Polyvinylpyrrolidone (PVP) is known to be sensitive to gamma-irradiation. Generally, an increase in average polymer molecular weight is observed when a PVP solution is exposed to 10-50 kGy of gamma radiation. Accordingly, the manufacturer does not recommend that the material be exposed to ionizing radiation for purposes of sterilization (KOLLIDON: Polyvinylpyrrolidone for the Pharmaceutical Industry, BASF Aktiengesellschaft, Feinchemie, D67056 Ludwigshafen).

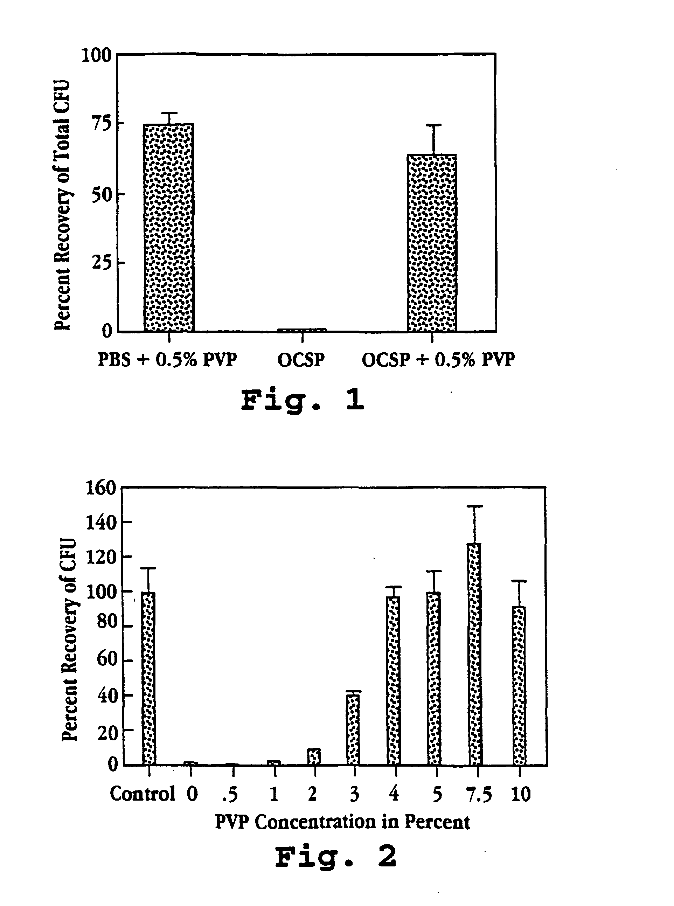

In experiments carried out in support of the present invention, it has been found that incubation of peripheral blood mononuclear cells (PBPC) in an OCS particle-based density gradient material sterilized by filtration (0.2 $\mu$m) or by heat-sterilization (autoclave, 15 min. at 120°C) does not significantly affect recovery of viable hematopoietic progenitor cells, as assessed by a functional cell assay, the colony forming unit (CFU) assay, which measures the potential of such cells to form hematopoietic colonies. However, when an OCS particle-based density gradient material was sterilized by exposure to gamma radiation (2.5-3.5 megaRads) according to standard methods, cells incubated in this medium according to the protocol described as Method 2 in Example 4 (incubation with OCS + 0.5% PVP for 30 minutes plus 10 minutes centrifugation time) exhibited reduced functional activity (to approximately 25 % of added CFU activity). The result was even more striking for cells treated according to the protocol described

as Method 4 in Example 4 where no culturing step was included. Here, cells were incubated for 30 minutes in an OCS particle-based density gradient material, OCS particles + 0.5% PVP or buffer + 0.5% PVP, followed by centrifugation for 10 minutes followed by 24 hours incubation in ISCOVES medium supplemented with 10% fetal cell serum. Inclusion of 0.5% PVP in the OCS particle suspension during preparatory sterilization and resulted in a recovery of about 65% of the CFU added (FIG. 1), while those cells incubated in an OCS particle-based density gradient material alone exhibited only about 1% of original functional activity. While this experiment used cells derived from PBMC, bone marrow- and cord blood-derived cells exhibit the same susceptibilities.

The amount of polylactam required to provide protection to cells was investigated in further experiments carried out in support of the present invention. FIG. 2 shows results from OCS particle-based density gradient material was supplemented with various concentrations of PVP prior to gamma irradiation (2.5-3.5 megaRads). In this study, PBPC cells were processed according to the protocol identified as Method 6 in Example 4 (incubation in presence of density gradient suspension and 10% fetal calf serum for 24 hours at room temperature). PVP in excess of 3% final concentration in the density gradient medium provided significant protection against irradiation-induced toxicity at the dose (2.5-3.5 megaRads) of radiation applied. Addition of PVP after sterilization of the medium did not provide a similar protective effect.

From the foregoing it will be apparent that the present invention includes an improved cell density gradient composition for use in separation of rare blood cells. The product composition is a silanized silica particle treated by the process of ionizing radiation in the presence of at least about 0.05% polylactam, and more specifically, between about 0.1 and 10% polyvinylpyrrolidone. Such a composition is particularly well suited for isolating rare blood progenitor cells, such as hematopoietic progenitor (CD34+) cells, that are sensitive to exposure to irradiated organosilanized colloidal silica.

D. Polylactam Improvement of Recovery of Rare Blood Cells from Density Gradients

In accordance with another aspect of the invention, it has been discovered that supplementation of a suspension of organosilanized silica particles with 0.05% (wt/vol) polylactam results in improved yields of rare blood cells in functional form. More specifically, inclusion of the polylactam prevents aggregation or clumping of the cells to provide a cell composition having a more uniform density profile and improved functional properties. In comparison to cells isolated in OCS density gradient material in the absence of a polylactam, such compositions are characterized increased recovery of rare cells in enriched fractions, and increased recovery of rare cells having clonogenic potential.

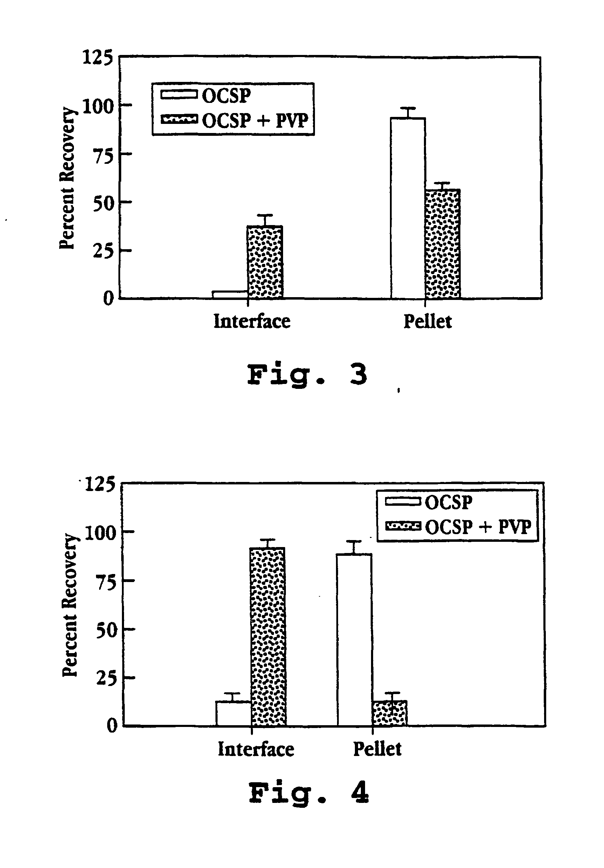

FIGS. 3 and 4 show the results of experiments in which PBPC isolated according to standard methods were loaded onto an organosilanized colloidal silica density gradient (OCS particle-based density gradient material, 1.0605 g/ml, 280 mOsm/kg $H_2O$, pH 7.4) and centrifuged according to the protocol detailed in Example 3. FIG. 3 shows that in the absence of PVP, most of the cells present in the starting material were recovered from the pellet. Visual inspection revealed that this pelleting of the cells appeared to be the result of aggregation or clumping of the cells. In contrast, when the OCS density material was supplemented with 0.5% PVP, a higher percentage of cells remained at the interface, as would be predicted based on the pre-determined density of these cells.

This observation was more pronounced when the same cell preparations were analyzed for the presence of hematopoietic progenitor cells (CD34+ cells) according to methods detailed in Examples 5 and 6 (FACS and CFU analysis). The results shown in FIG. 4 show that in the absence of PVP, about 90% of the CD34+ cells migrated to the pellet. The presence of 0.5% PVP in the density gradient suspension reversed this pattern. Here, most of the CD34+ cells remained at the interface, as is desirable for purposes of isolation and enrichment of such cells for transplantation and other clinical purposes.

III. Methods of Isolating Specific Cell Types

In view of the foregoing, it can be appreciated that the present invention includes not only a density gradient composition that improves recovery of rare blood cells, but also a method of isolating functional rare blood cells, and particularly cells such as hematopoietic progenitor cells (e.g.. CD34+ cells), dendritic cells, natural killer cells, natural suppressor cells, and the like from a cell mixture.

Generally, hematopoietic progenitor cells can be isolated from bone marrow, cord blood, or from peripheral blood samples derived from a subject, particularly a human subject. It is desirable to first mobilize the cells in the subject by administering agents such as granulocyte colony stimulating factor (G-CSF), VP-16, or a combination of the two factors, according to protocols well known in the art. Following such treatment, the patient is subjected to apheresis to collect PBPC or to bone marrow aspiration to collect bone marrow.

According to the present method, the collected cells are layered onto an density gradient material, and preferably, an organosilanized colloidal silica particle-based cell separation density gradient suspension that has a specific density that is approximately equal to that of the cells to be isolated. The medium preferably includes at least about 0.05% PVP, and more preferably, at least about 0.5% PVP. The suspension is then centrifuged to pellet cells having a specific density that is greater than the specific density of the selected hematopoietic cell. Such a density gradient composition also has the advantage of being capable of being sterilized by methods described above.

The foregoing method can also be enhanced by adding to the initial cell mixture a suspension that includes an organosilanized silica particle to which is attached a cell antigen-specific binding molecule for negative or positive selection. As an example of negative selection, the antigen-specific binding molecule binds an unwanted cell type in the cell mixture to cause the cell to migrate to the pellet. As an example, it is sometimes desirable to deplete T-cell lymphocytes from a cell mixture, such as a hematopoietic stem cell mixture, prior to reintroducing the selected cells to the body. Such T-cell lymphocytes can be depleted, for example, by treating the cell mixture with beads to which a T-cell lymphocyte-specific antigen is bound. The beads are then separated from the desired cell mixture by centrifugation. Examples of T-cell lymphocyte specific antigen binding reagents that can be bound to beads include anti-CD3, anti-CD4 and anti-CD8 mouse monoclonal antibodies.

Any tubes suitable for use in centrifugation may be used for the practice of the invention. In a preferred embodiment of the present invention, the cell-trap centrifugation apparatus described in WO 96/07097 will be used for the density separation of CD34$^+$ cells.

A. Isolation of Hematopoietic Progenitor CD34$^{\pm}$ Cells

Co-owned PCT application PCT/95/11169 publication WO 96/07097 describes studies in which "PERCOLL$^®$ was used at physiologic osmolality of 280 $\pm$ 10 mOsmlkg $H_2O$ and pH of 7.4 as the density gradient material to isolate CD34$^+$ cells from apheresed blood. According to the invention described therein, at this osmolality, CD34$^+$ cell recovery was optimal at a specific density of 1.0605 gr/ml.

In studies carried out in support of the present invention, bone marrow cells were collected by aspiration, according to standard clinical procedures, from healthy donors were obtained from the Bone Marrow Transplantation Laboratory of the Stanford University School of Medicine, Palo Alto, CA. CD34$^+$ cells were enriched from the bone marrow cell fraction by centrifugation over an OCS gradient having a specific density of 1.0685 gr/ml at 280 mOsm/kg $H_2O$, pH 7.4, prepared and formed in accordance with the methods of the present invention, as described in Example 10.

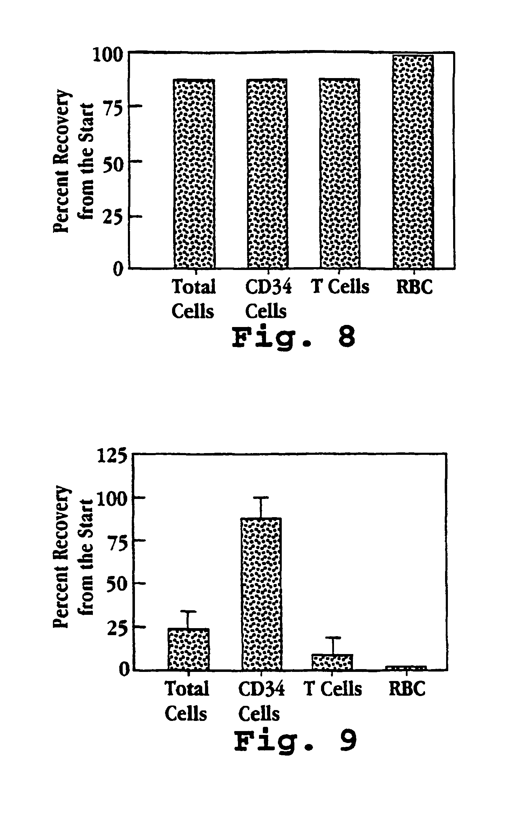

FIGS. 8 and 9 summarize results of isolation studies on bone marrow cells processed on the 1.0685 gr/ml OCS suspension. FIG. 8 shows percent recovery of total cells, CD34$^+$ cells, T lymphcytes and red blood cells (RBC) from the entire gradient (interface + pellet). FIG. 18 shows the percent recovery of the same cell fractions from the interface of the gradient following centrifugation. Data presented in both figures were calculated by dividing each fraction by the total number of cells initially loaded on the gradient.

From FIG. 8, it can be seen that over 90% of cells initially loaded on the gradient were recovered from the interface and pellet. Since the loss of CD34$^+$ cells was comparable to the approximate loss of total cells (10%), there was no preferential loss of these cells.

FIG. 9 shows that while only 26 $\pm$ 10% of total cells was recovered from the interface, this fraction contained 91 $\pm$ 9% of the CD34$^+$ cells, 10% of T lymphocytes, and essentially no red blood cells. These studies demonstrate substantial enrichment of CD34$^+$ cells, coupled with depletion of T lymphocytes, by the density gradient procedure of the present invention. As discussed in the following section, these characteristics recommend the preparation for use in allogeneic stem cell transplantation.

B. Natural Suppressor Cells

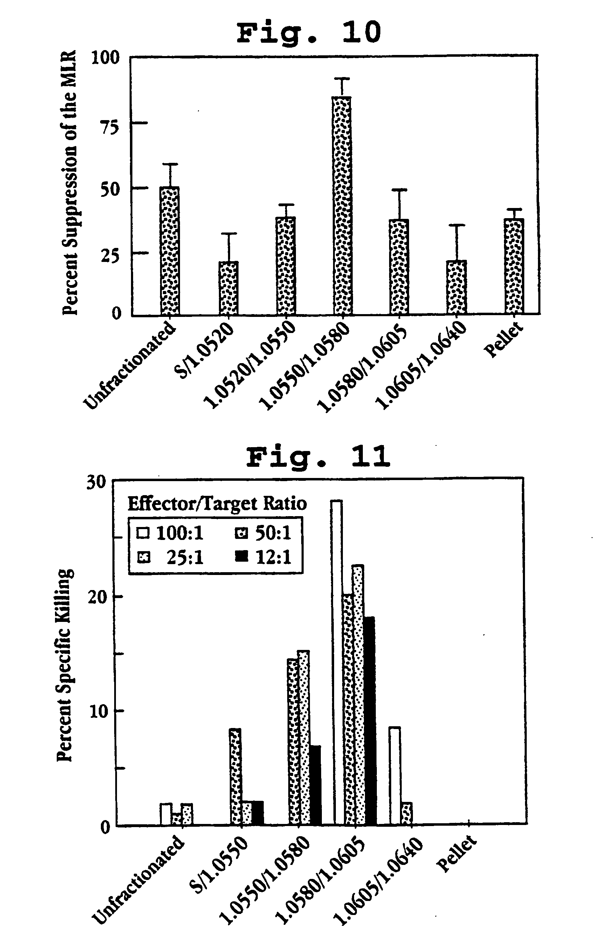

*In vitro* studies have shown that human bone marrow contains low density cells that block *in vitro* alloresponses in the mixed lymphocyte reactions (MLR). Based on the fact that this suppressive activity was HLA non-restricted, it was referred to in the literature as natural suppressor (NS) activity. A "PERCOLL$^®$ density gradient was adjusted to a density of 1.0605 $\pm$ 0.0005 gr/ml to test for its ability to enrich cells with NS activity. Apheresed blood samples from lymphoma patients and from normal individuals that received G-CSF treatment were centrifuged on a discontinuous five layer gradient, and the interfaces and pellet were screened for their potential to suppress the mixed lymphocyte culture. FIG. 10 shows that cells with NS activity had a density equal or lighter than 1.0605 gr/ml. Consequently, more than 90% of the NS activity was present in the final cell preparation after centrifugation on a 1.0605 gr/ml gradient.

C. Natural Killer Cells

Natural Killer (NK) cells have been shown to kill autologous tumor cells. From a clinical perspective, it may be beneficial to have increased numbers of NK cells in the transplant to reduce tumor relapse. In experiments carried out in support of the present invention the density of the NK cells was determined on a discontinuous five-layer "PERCOLL$^®$" gradient. NK cells also showed a density equal to or lighter than 1.0605 gr/ml. Consequently, more than 90% of NK cells was present in the final cell preparation after centrifugation on a 1.0605 gr/ml gradient, as shown in FIG. 11.

D. Enrichment of Dendritic Cells and Cytotoxic T Lymphocytes

Dendritic cells (antigen presenting cells) are cells that are derived from blood fractions. These cells act in vivo to present antigen to T lymphocytes to induce a primary cytotoxic T lymphocyte (CTL) response. These cells have clinical utility for use in inducing or enhancing cellular immune responses.

According to another aspect of the present invention, dendritic cells and cytotoxic lymphocytes are isolated using defined density gradients, in a three step process. This process includes centrifugation through three different density gradient solutions, as detailed in Example 12. In accordance with the present invention OCS density gradient material having densities of 1.0770, 1.0720 and 1.0610 or 1.0720, 1.0610, and 1.0565 gr/ml, each at pH 7.4 and an osmolality of 280 mOsm/kg $H_2O$ are used in the three steps, which are conveniently, but not necessarily carried out in a cell-trap tube. Practitioners will recognize that equivalent densities of other media can be used as will. For example, step one can be carried out in Lymphoprep (Nycomed Laboratories, Oslo, Norway) or in Ficoll equivalent PERCOLL® (density 1.0770, 310 mOsm/kg $H_2O$, pH 7.4); step two can be carried out in a PERCOLL® solution (density 1.0650, pH 7.4, 300 mOsm/kg $H_2O$; and step three can be carried out in a PERCOLL® solution having a density of 1.0800 gr/ml, pH 7.4, 540 mOsm/kg $H_2O$, or in a PERCOLL® solution having a density of 1.0550 gr/ml, 290 mOsm/kg $H_2O$, pH 7.4). However, the foregoing densities determined using OCS particle based cell separation and the enrichment methods of the present invention, are advantageous in that they are carried out at physiological osmolalities, rather than under the hyperosmotic conditions previously reported.

E. Density Adjusted Cell Sorting (DACS)

The present invention also provides for purging from the bone marrow cells or other progenitor cell source contaminating tumor cells or other undesirable cells. For purposes of purging, the density of a given tumor cell is determined by centrifuging a tumor containing sample on a discontinuous density gradient ranging from 1.0490 to 1.0640 gr/ml. The majority of undesirable, non-tumor cells represent cells of the immune and hematopoietic system and have a density in the range of 1.0610 to 1.0770. The density of tumor cells generally falls within the 1.0490 to 1.0580 gr/ml density range. The density of the cell separation medium is determined to an accuracy of within $\pm$ 0.0005 gr/ml, preferably $\pm$ 0.0002 gr/ml of the specific gravity of the desired cells. Thus tumor purging can be accomplished by a two step process, in which the desired cells are first pelleted through a cell separation medium capable of retaining tumor cells. Alternatively, the purging technique can be used to remove tumor cells in a one-step process, according to the methods described below.

Experiments carried out in support of the present invention demonstrated that CD34+ cells were enriched using the DACS procedure in conjunction with the density method of the invention, by removing contaminating cells with an anti-CD45 mAb coupled to a heavy carrier (such as magnetic beads or aminopropyl glass beads). The total cell number was reduced 82% and the CD34 yield was around 40%. CD34 purity was increased from 2% to approximately 20%. Since the anti-CD45 antibody removed also some of the CD34+ cells, it is appreciated that this method could be improved by using a mixture of other antibodies to deplete non-stem cells.

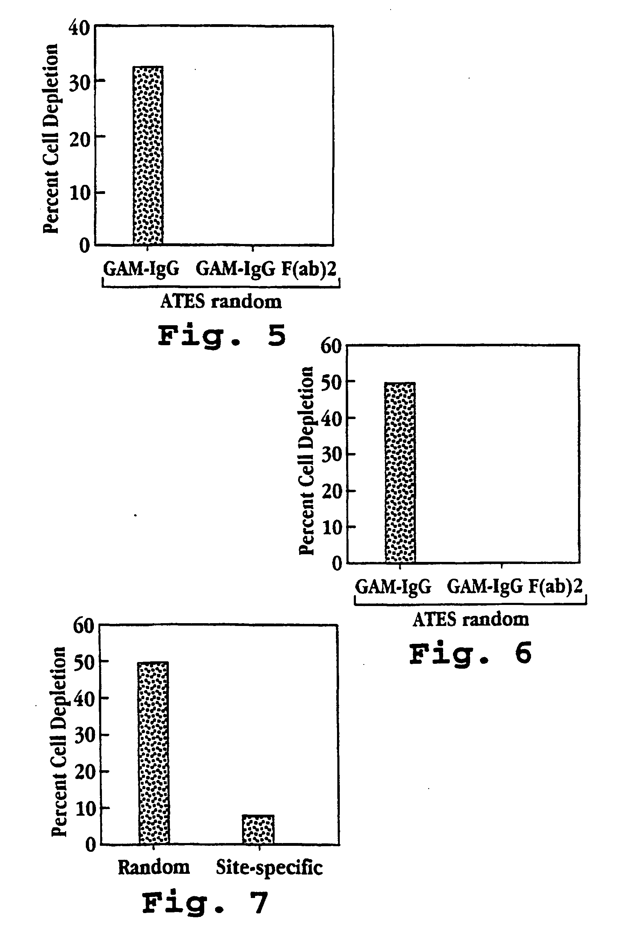

To examine non-specific cell losses during DACS, experiments were performed using buffy coat peripheral blood mononuclear cells (PBM) and DACS beads in the absence of monoclonal antibodies. As detailed in Example 10, DACS beads were made using either intact GAM-IgG or F(ab)$_2$ GAM-IgG to test the theory that Fc receptor mediated binding of cells to IgG-coated DACS beads contributed to non-specific depletion. The results in FIG. 5 demonstrate that there was no non-specific depletion of cells when GAM-IgG F(ab)$_2$ coated DACS beads were used compared to DACS beads with intact GAM-IgG. DACS beads coated with GAM-IgG, linked via the glycosylated site on the Fc region, also demonstrated reduced non-specific depletion of cells when compared with DACS beads with GAM-IgG in a random orientation. These results demonstrate that removing or blocking the Fc region of the GAM-IgG leads to a reduction in non-specific depletion of cells.

The foregoing results show that Fc Receptor (FcR)-mediated binding of cells to DACS beads may be responsible for some of the non-specific depletion of CD34+ cells observed during DACS treatment. This is further illustrated by the site-specific coupling which results in a reduction of non-specific loss of cells due to blocking of the glycosylated areas of the Fc portion of the immunoglobulin molecule, as described above. However the use of BSA-coated DACS beads has demonstrated that FcR-mediated binding is not usually responsible for all the non-specific depletion of cells. Ionic interactions between the cell and the bead surface are also likely to be involved. To investigate this possibility, DACS beads coated with PEG sandwiched between the bead surface and the GAM-IgG were made. This was done to exploit the ability of PEG to reduce ionic interactions between proteins and charged surfaces. In an experiment comparing PEG-GAM-IgG-coated DACS beads with DACS beads with GAM-IgG in the absence of PEG, it was discovered that the use of PEG is associated with a reduction in non-specific depletion of cells.

Reduction in non-specific depletion by modification of the DACS bead coating is particularly important in the context of the negative enrichment of rare cells such as CD34+ cells, NK cells, natural suppressor cells, dendritic cells, fetal nucleated cells, and the like, where it is important to maximize the yield of cells. For example, experiments to define the non-specific loss of CD34$^+$ cells demonstrated that the use of F(ab)$_2$ coated DACS beads lead to a reduction in the non-specific loss of CD34$^+$ cells (FIG. 6). Likewise, using DACS beads coated with GAM-IgG1 bound via the glycosylation site of the Fc region also reduced non-specific depletion of CD34$^+$ cells when compared with DACS beads with GAM-IgG in a random orientation (FIG. 7).

E. Depletion of T-cell Lymphocytes

Graft versus host disease (GvHD) is induced by T lymphocytes that are present in donor allografts, though complete removal of such cells also results in failure of the graft and tumor relapse. Thus the presence of a limited number of T lymphocytes may be required for successful allogeneic transplant. In experiments carried out in support of the present invention, DACS beads were used to deplete T lymphocytes from apheresed PBSC preparations from G-CSF mobilized donors. T lymphocytes were depleted from a mobilized PBSC preparation by adding to the preparation of mixture of anti-CD3, anti-CD4 and anti-CD8 mouse monoclonal antibodies, followed by goat anti-mouse-IgG DACS beads, and centrifugation through an OCS gradient having a density of 1.0605 gr/ml at 280 mOsm, pH 7.4. T lymphocytes were monitored by reaction with anti-CD2 monoclonal antibodies, in order to avoid possible artifacts associated with using antibodies that recognize the same epitopes on the differentiation antigens.

The foregoing DACS T lymphocyte depletion method resulted in a 97% reduction in T lymphocytes with only a 70% reduction in the yield of CD34$^+$ cells. Sixty percent of the CD34$^+$ cells were retained in the interface after DACS treatment. The results demonstrate that the combined cell enrichment and DACS method specifically reduces T lymphocyte content of allograft material by 97% with less than 40% loss of CD34$^+$ cells. Such a preparation is suitable for preventing GVHD in transplant material.

F. Enrichment and Purging of Tumor Cells

1. Enrichment of Tumor Cells for Diagnosis

Breast tumor cells are used to exemplify the enrichment methods of the present invention. To enrich for such cells, a gradient should be adjusted to a density of 1.0490 to 1.0580 $\pm$ 0.0005 gr/ml, depending upon the specific type of breast tumor cell, a physiologic osmolality of 270-290 mOsm/kg H$_2$0 and physiologic pH 6.8-7.8. In a specific embodiment by way of examples, breast tumor cells are directly loaded into a cell-trap centrifugation tube containing an appropriate cell separation medium, such as OCS-particle-based cell separation medium or "PERCOLL$^®$" solution filled to a level above the constriction.

In the specific examples described herein, successful enrichment of tumor cells was carried out on "PERCOLL" density gradient material which had been adjusted to the specific density of between 1.0490 to 1.0580 $\pm$ 0.0002 gr/ml, depending upon the specific type of breast tumor cell, osmolality of 280 mOsm/kg H$_2$0 and pH 7.4. The density of the "PERCOLL$^®$" solution may be adjusted on a densitometer to precisely define its accuracy.

The cells enriched by the methods described above may subsequently be examined for the presence of breast tumor cells. The resultant yield of isolated or enriched cells from the cell separation methods of the present invention may be used for diagnostic purposes, *e.g.* morphological, molecular, biochemical or immunophenotypic assays. For example, DNA may be prepared from the collected cells and subjected to polymerase chain reaction (PCR), as described under the tumor cell purging section below, or the collected cells may be assessed morphologically thereby avoiding the use of invasive and expensive surgical procedures heretofore relied upon for such a determination.

Various breast tumor antigens and breast tumor markers are known to those of skill in the art or are commercially available including but not limited to cathepsin D, EGF-R ,estrogen receptor, Ki-67, progesterone receptor and TGF-$\alpha$. Antibodies directed to these antigens or markers may be used to assess the tumor type of collected cells.

A major advantage of the methods described herein is that a large volume of complete blood may be directly placed on the density gradient. Peripheral blood may be collected in anti-coagulant-containing tubes or by apheresis or leukopheresis. Complete blood does not need to be processed or diluted prior to centrifugation. However, since the methods enrich breast tumor cells based on their specific buoyant density, it is important that the cells are subject to separation within a relatively short time after their collection from an *in vivo* source because the density of the cells changes according to their culture or storage conditions. Therefore, in order to obtain optimal enrichment of breast tumor cells from blood, it is preferred that the blood samples are used within 48 hours after their collection. Most preferably, blood samples should be subjected to density gradient centrifugation within several hours of collection.

2. Purging of Tumor Cells

Tumor cell purging is conveniently carried out using the tumor cell enrichment methods discussed above, or more preferably, the DACS augmented cell separation methods described above. By way of example, when radioactively labeled breast tumor cells were mixed with a peripheral blood buffy coat, up to 80% of were retained by centrifuging the mixture on a gradient having a specific density of 1.0580 gr/ml and an osmolality of 280 mOsm/kg $H_2O$. In addition, only a small fraction (< 10% of initial cell number) of non-tumor cells were found in the collected tumor fraction.

Co-owned PCT application PCT/95/11169 publication WO96/07097 describes enrichment of breast tumor cells according to the methods of the invention. This method may also be used in conjunction with the DACS procedure described herein. For example, complete blood can be directly incubated with carrier particle- coated-anti-CD45 antibodies which react with most leukocytes. Since breast tumor cells do not react with anti-CD45 to any significant degree, the vast majority of the non-red blood cells, leukocytes, and other cells are rendered heavier than the density material and pellet during centrifugation, while the breast tumor cells remain in the upper compartment. A variety of other cell type-specific binding agents may be used to target specific cell types in the blood, as discussed in the previous sections.

Alternatively, according to a preferred purging procedure, the positive selection DACS procedure may be used to cause tumor cells to be heavier than their normal densities, so that they are pelleted during centrifugation. Cell type-specific binding agents useful in the positive selection procedure include, but are not limited to antibodies to breast tumor antigens and antibodies to breast tumor markers, *e.g.* HER2/Neu, CA 15-3, CA 549, cathepsin D, EGF-R, estrogen receptor, Ki-67, progesterone receptor, and TGF-$\alpha$. Many of these antibodies are commercially available.

The cells enriched by the methods described herein can subsequently be examined for the presence of breast tumor cells. The resultant yield of isolated or enriched cells from the cell separation methods of the present invention may be used for diagnostic purposes, *e.g.* morphological, molecular, biochemical or immunophenotypic assays. For example, DNA may be prepared from the collected cells and subjected to polymerase chain reaction (PCR) or the collected cells may be assessed morphologically thereby avoiding the use of invasive and expensive surgical procedures heretofore relied upon for such a determination. Alternatively, tumor cells can be identified by the presence of tumor cell markers known to those of skill in the art, as described above. Antibodies directed to these antigens or markers may be used to assess the tumor type of collected cells.

3. Tumor Purging of Peripheral Blood Samples

The presence of tumor cells in stem cell grafts and the inability to specifically deplete tumor cells from the bone marrow of non-Hodgkins lymphoma patients may be an important indicator in predicting disease relapse after autologous transplantation. Cell separation methods in accordance with the present invention can be applied to remove tumor cells from peripheral blood products prior to re-introduction of such products into the patient.

In studies carried out in support of the present invention, the human B-cell lymphoma cell line SU-DHL4 was used to model tumor cell contamination of peripheral blood. This cell line contains a reciprocal translocation between the long arms of human chromosomes 14 and 18, resulting in a juxtaposition of the bcl-2 proto-oncogene to the joining region of the immunoglobulin (Ig) heavy chain gene. This translocation, which leads to elevated levels of the bcl-2 gene product, is found in 85% of follicular small cleaved cell lymphomas and 25% of diffuse large cell lymphomas. The translocation breakpoint in the bcl-2 gene is located in either the major breakpoint region (MBR) spanning approximately 150 bp in the 3' untranslated region (UTR) of the bcl-2 gene or the 500 bp minor cluster region (MCR) located approximately 20 kb downstream of the bcl-2 gene. Using translocation-specific primer pairs and quantitative PCR by limiting dilution analysis techniques known in the art, depletion of SU-DHL4 cells in DACS purging experiments was monitored.

Likewise, human SK-BR3 adenocarcinoma cell line is used to model breast cancer cell infiltration of peripheral blood. This cell line reacts with the mouse anti-human Her2/Neu monoclonal antibody. Prior to being spiked into normal buffy coats, the SK-BR3 cells-were pre-loaded with calcein AM, a fluorogenic substrate so that they could be enumerated by Fluorescence Activated Cell Sorting (FACS) analysis. Details of the experimental methods used in carrying out tumor purging experiments are provided in the Examples.

A PCR assay was used to monitor tumor cell levels in the blood cell mixtures. Initially, mixtures of genomic DNA from SU-DHL4 and a (14; 18) translocation-negative human cell line (K562) were used as starting templates to assess the level of sensitivity of the PCR assay. Agarose gel electrophoresis was used to separate PCR products from either one round or from two-rounds of amplification, as shown in. PCR products specifically derived from SU-DHL4 DNA were identified on the gel. The results of these preliminary tests showed that a nested PCR assay can detect approximately 1 SU-DHL4 cell per 150,000 cells.

In addition, genomic DNA was prepared from frozen aliquots of apheresed blood of 24 non-Hodgkin Lymphoma (NHL) patients who received chemotherapy and G-CSF, according to standard methods known in the art. Nested PCR products using primer-pairs specific for the major breakpoint region (MBR) translocation were found in 4 patients, demonstrating that this assay can detect cells carrying the translocation from authentic clinical samples.

Further experiments were carried out to validate the use of PCR analysis under conditions of limiting dilution for quantitation of tumor cell frequency in a sample. Replicate PCR reactions were performed using known amounts of input SU-DHL4 genomic DNA. Nested PCR primer-pairs for t (14;18) (a target sequence present once per SU-DHL4 cell) and p53 (a target sequence present twice per SU-DHL4 cell) were used. The observed frequency of positive PCR reactions demonstrated that experimental frequencies are similar to frequencies expected based on Poisson statistics. Thus, PCR using limiting dilution analysis was demonstrated to provide an accurate, quantitative method for monitoring tumor cells.

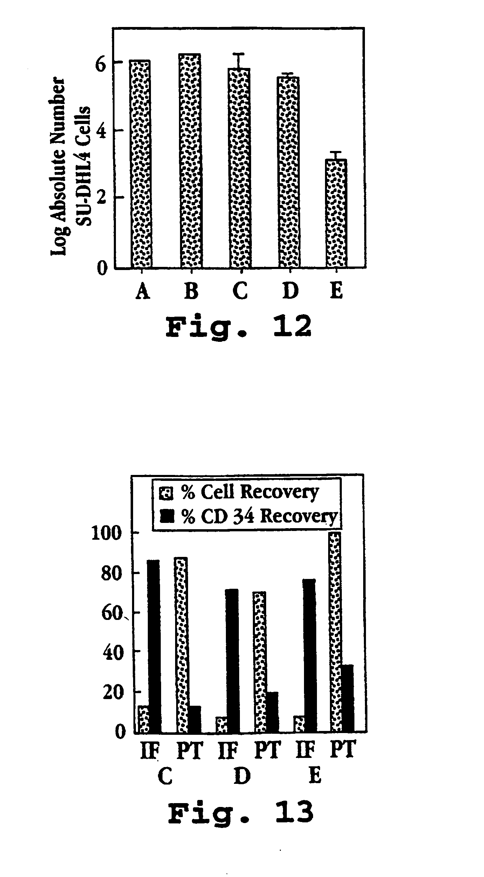

To model tumor cell contamination of PBPC, samples of apheresed blood from G-CSF mobilized patients were seeded with a known number of SU-DHL4 cells. This spiked material was used to assess tumor cell removal by DACS. This method involves incubation of the blood with mouse monoclonal antibodies specific to surface antigens expressed on the target cell (CD9, CD10, CD19, CD20). This step was then followed by incubation with goat anti-mouse Ig coated high-density microspheres (DACS beads). The sub-population of cells bound to these microparticles was then separated from unbound cells by centrifugation through an OCS suspension supplemented with 0.5% PVP at a specific density of 1.0605 gr/ml and sterilized by gamma irradiation. Cells were recovered from the interface (IF) and from the pellet (PT) after gradient centrifugation and were quantitated by gel electrophoresis, as detailed in Example 5. The results of a representative purging experiment are shown in FIG. 12 and FIG. 13.

FIG. 12 shows the number of spiked SU-DHL4 cells in the; A) unfractioned sample (determined by visual count), B) unfractioned sample, C) interface without DACS, D) interface after using GAM-IgG DACS beads, and E) interface after using GAM-IgG DACS beads and a cocktail of anti-B cell mAbs. The number of SU-DHL4 cells in B, C, D and E was determined by quantitative-PCR.

FIG. 13 represents the recovery of CD34$^+$ cells in the interface (IF) and pellet (PT) of C, D and E. Tumor cell depletion was quantitated by PCR limiting dilution analysis for samples B-E, as illustrated. The recovery of CD34$^+$ hematopoietic stem cells was monitored by three-color flow cytometry. Recovery of total cells and stem cells are shown relative to the recovery of cells from the interface. A single purging cycle using an antibody cocktail resulted in specific 2-3 log depletion of SU-DHL4 cells. The recovery of CD34$^+$ cells after DACS was greater than 70% and comparable to the number of CD34$^+$ cells recovered from the interface in the absence of DACS. Peripheral blood mononuclear cells are reduced by 70-80% by the density centrifugation with or without DACS.

In another model for tumor cell contamination, SK-BR3 cells were seeded into normal buffy coats at a frequency of 2%. The calcein AM labeled cells tumor cells were removed from the cell suspension by centrifugation on an OCS density suspension having a specific density of 1.0605 gr/ml with DACS using anti-human Her-2/Neu Mabs. The depletion vas determined by FACS analysis. The calcein AM labeled cells were gated and their percentage determined using the LYSYS II program. Whereas the SK-BR3 cells were not depleted from the buffy coat after processing on OCS suspension in the presence or absence of DACS beads, they were almost completely removed from the cell mixture when using DACS beads in combination with the breast cancer specific anti-Her2/Neu mAb. These data show that DACS treatment reduces tumor cell contamination by 1 to 2 logs (10-100-fold) when the FACS percentage is back calculated to the initial tumor cell number spiked in the sample.

In summary, the foregoing exemplary model experiments using a human B-cell lymphoma cells and quantitative PCR demonstrate that a 2-3 log reduction in tumor cells can be achieved in one round of DACS while retaining the majority of CD34$^+$ hematopoietic stem cells. Using a human adenocarcinoma cell line SK-BR3 and FACS analysis, a 1-2 log reduction in tumor cells was achieved. More generally, it is the discovery of the present invention that this method can be used to purge blood or therapeutic enriched cell populations of unwanted tumor cells.

IV. Sterilized Cell Separation Kit

An important application of the discovery of the present invention is a sterile kit for cell separation. Such a kit is particularly useful for clinical cell separations. Such a kit will commonly include a centrifugable container and a density gradient material for separating cells, which has been sterilized as a filled unit by exposure to ionizing radiation. The centrifugable container will commonly be a consumable, disposable unit formed from plastic, such as polypropylene, polycarbonate, polysulfone, polymethylpentene, polyethylene, polyallomer, polystyrene and the like. However, as discussed below, the advantages of sterilizing the kit as a pre-filled unit also makes this procedure desirable for kits that include tubes formed from other materials. In a preferred embodiment, the container will be closed, as illustrated below, to facilitate aseptic collection of starting material and preservation and manipulation of the material in a sterile condition.

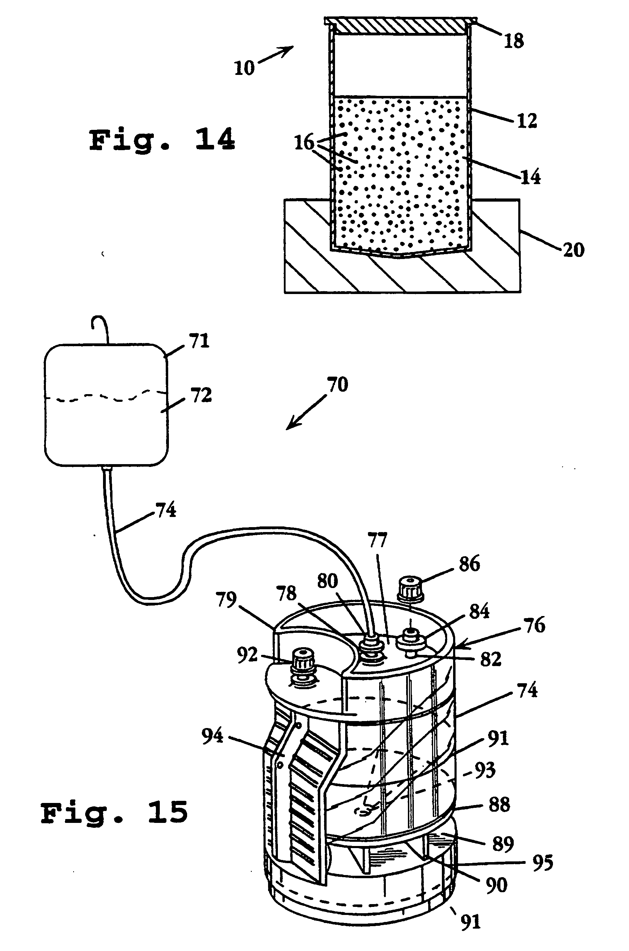

FIG. 14 illustrates a kit 10 formed in accordance with the present invention. As illustrated, kit 10 includes centrifuge tube 12 shown here filled with density gradient material 14. The density gradient material is formed by a suspension of organosilanized silica particles, such as particles 16. Also included in the density gradient material is at least 0.05% PVP, and preferably between about 0.1% and 10% PVP to reduce toxicity to rare blood cells isolated in the kit, thus improving yield of such cells in the kit.

The container and the density gradient contained within are sterilized by ionizing radiation, such as gamma irradiation or E-beam. The resulting kit may also include a protective cover, such as cap 18 to protect the contents of the tube from contamination after sterilization. The kit may also include a stand, such as tube-holder 20, to prevent displacement of tube contents during shipment and storage.

Kits as described above can be prepared according to the specifications required for separation of a number of cell types. The size of tube used, as well as its structural and chemical resiliency can be varied within the confines of the present invention for use in ultracentrifugation as well as in relatively low-speed centrifugation applications as described herein. Such modifications will be apparent to persons skilled in the art.

Similarly, the kit may be designed for different cell types by varying the composition of the density gradient material. Example 9 describes a kit that is particularly formulated for isolation of CD34[+] hematopoietic progenitor cells from PBPC. In the embodiment described, the OCS particulate density gradient suspension is adjusted to a specific density of 1.0605 g/ml and an osmolality of 280 mOsm/kg $H_2O$. This density is approximately equal to the density of the desired CD34[+] cells, so that upon centrifugation, these cells will "float" at the interface between the loading material and the density material present in the tube.

It is appreciated that enrichment of other rare blood cell types, such as dendritic cells, cytotoxic T cells, natural killer cells, natural suppressor cells and fetal nucleated cells can be achieved by adjusting the specific density and osmolality of the density gradient material. Such adjustment can be made so that the desired cells will then either float or pellet, according to the density of the desired cells and the relative density of unwanted cells present in the cell mixture from which the cells are to be isolated.

A preferred centrifuge tube configuration for use in the kit and method of the invention is one that has a constriction member disposed within the tube, forming a "cell-trap." Such a tube is described in co-owned PCT application PCT/95/11169 (publication number WO 96/07097). A modified form of this tube is illustrated as part of a closed system in FIG. 14 herein, described below.

In the cell-trap tube, the constriction member is positioned and constructed to retain fluid in the bottom portion of the tube below the constriction member when the tube is inverted. The tube also includes cell-separation medium that is contained in the bottom portion of the tube and which extends above the constriction member to a level above the opening formed by the constriction member. Cells that are captured at an interface between the cell-separation medium and a lower-density medium, after centrifugation, are discharged with the lower-density medium when the tube is inverted. This facile means of collection of cells is particularly convenient in the context of the present invention, where minimal manipulation of the cells is desirable.

It is also appreciated that, for clinical applications, the kit can be configured as a "closed system" to restrict access of external pathogens to the biological materials, as described herein.

FIG. 14 shows a centrifugeable container having a constriction member incorporated into a closed system. As illustrated, closed system 70 includes a reservoir 71, shown as sterile bag, containing blood 72 previously collected by known techniques, is connected by sterile connecting tubing 74 to centrifuge container 76, illustrated as a style centrifuge tube having a closed top 77. The closed top will have at least one, and preferably at least two entry ports, useful for introduction and removal of sample, and for venting, as described below. In the embodiment shown, solid ridge 79 protruding upward from closed top 77 is included to form a protective barrier for the entry ports, and as an attachment point for a protective, removable lid for the apparatus that serves to reduce potential contamination during shipping and storage.

With further reference to FIG. 14, tubing 74 is attached to container 76 through entry port 78, adapted with fitting 80, which may be any type of locking tip adapted for sterile connection, for example, a Luer-Lock™ syringe connector. Alternatively, fitting 80 may be a sterile septum adapted for connection with sterile fluid bags and tubes, for example a SAFSITE™ small wire extension set with reflux valve and Spin-Lock™ adaptor available from Burron Medical Inc., Bethlehem, Pennsylvania. To facilitate fluid flow into centrifuge tube 76, the bucket contains air vent entry port 82. As shown, air filter 84 is attached to entry port 82 to prevent contamination.

In accordance with a preferred embodiment of the invention, container 76 includes constriction member 88. Although a number of configurations are possible, as illustrated, constriction member 88 is funnel shaped on its upper surface. As illustrated, constriction member 88 is formed integrally with container 76 forming an indentation 89 on the outer surface of the tube. The constriction member is supported by supports 90 to prevent compression during centrifugation. The container also contains OCS particle density gradient material 91 disposed in the tube both above and below constriction member 88. The entire system as illustrated is sterilized by gamma irradiation or E-beam according to standard methods.

An added feature of container 76 is entry port 92. This entry port communicates via closed fluid channel 94 with the bottom portion 95 of container 76. The entry port and channel are used to fill the lower portion of the tube 95, for example, with density gradient cell separation medium prior to sterilization. Alternatively, the port and channel may be used to remove materials, including cell pellet materials, from the bottom of the tube following centrifugation. This feature of the tube is neither required by nor restricted to the closed system context in which it is illustrated.

Fluid flow from reservoir 70 into container 76 can be initiated by applying suction on air vent entry port 82, or it may be initiated by other means known in the art, including gravity. The rate of flow is adjusted, either by altering the pressure head between the two containers or by regulating a valve positioned in the tube or at the entry port at a point between reservoir 70 and entry port 78. Flow rate is optimally regulated to fill or partially fill the upper portion of container 76, above the level of the density gradient solution. When sufficient fluid sample has entered the tube, flow can be terminated by any of a number of means known in the art, such as regulation by a valve or by lowering of pressure head. The tubing is then removed from the bucket, port 78 is sealed, and the closed container is subjected to centrifugation as described above.

In another preferred embodiment that is particularly useful in carrying out the method of the present invention, the constricted tube may take the form of a centrifugeable syringe, such as the centrifuge syringe described in co-owned PCT application PCT/US95/11162 (WO 96/06679). This embodiment is well suited for use in a closed system as described above.

Another configuration of the device that is suitable for use in the invention is a centrifugeable bag. Such a configuration is particularly suitable for use in processing clinical blood samples which can be collected directly into the bag.

The following examples illustrate, but are in no way intended to limit the present invention.

## EXAMPLES

## Materials

### A. Density Gradient Materials

Organosilanized colloidal silica particles (size range: 15-30 nm) used in the examples below were prepared according to the methods described in U.S. Patent 4,927,749 using gammaglycidoxypropyl-trimethoxysilane (GPMS). Polyvinylpyrrolidone (PVP-10) was obtained from Sigma (St. Louis, MO).

B. Monoclonal Antibodies

Monoclonal antibodies (mAb) directed against surface antigens specific for hematopoietic progenitor cells (anti-CD34; anti-HPCA-2) and leukocytes (anti-CD45; anti-HLE-1) were obtained from Becton Dickinson, Inc. (San Jose, CA). The different monoclonal antibodies were directly labeled with fluorescein isothiocyanate (FITC) or phycoerythrin (PE). PE labeled isotype control IgG1 polyclonal antibodies were obtained from Becton Dickinson, Inc. (San Jose).

## Example 1

### Preparation of Density Gradient Solution

The OCS particle-based density gradient material prepared as described under "Materials" above was diluted in phosphate buffered saline (PBS) to provide a suspension having a defined osmolality and specific density at pH 7.4, as indicated.

The OCS particle-based density gradient material was diluted in water to the approximate specific density required. Solid salts were added to provide a final physiological salt solution (physiological saline or Dulbecco's phosphate buffered saline; Gibco). The suspension was supplemented with different final concentrations (0%-10% w/w) of polyvinylpyrrolidone (PVP-10) by adding the solid powder adjusted for the density of the final suspension. The diluted suspension was finally sterilized by ionizing irradiation (gamma irradiation and E-beam, 2.5-3.5 megaRads; Isomedix, Morton Grove, IL), autoclaving (15 min. at 120°C) or filtration through a 0.2$\mu$m (micron) filter according to standard methods known in the art and used at room temperature.

## Example 2

### Preparation of Hematopoietic Progenitor Cells

A. Blood Cell Mixtures -

Peripheral blood mononuclear cells (PBPC) were collected by apheresis from non-Hodgkins lymphoma (NHL), Hodgkins lymphoma (HL) and breast-cancer patients at the Bone Marrow Transplantation Laboratory at the Stanford University School of Medicine, Palo Alto, California, USA. PBPC were mobilized in NHL patients by treatment with cyclophosphamide (4 g/m$^2$, intravenously) followed by granulocyte colony stimulating factor (G-CSF) (10 $\mu$g/kg, intravenously, daily). PBPC were mobilized in breast-cancer patients by treatment with etoposide (VP-16; 2g/m$^2$, intravenously) followed by G-CSF (10 $\mu$g/kg, intravenously, daily). PBPC were mobilized in HL patients by treatment with G-CSF alone. The mobilization protocols used are standard protocols known in the art.

Twenty-four hours following the final injection, each patient was subjected to apheresis. PBPC were collected from the apheresed blood according to standard methods.

B. Bone Marrow Cell Mixtures

Bone marrow aspirate cells were layered slowly on an OCS suspension prepared as described above to a density of 1.0685 gr/ml at an osmolality of 280 mOsm/kg $H_2O$. A maximum of $2 \times 10^9$ cells was layered and processed per centrifuge tube. The tube was centrifuged for 30 minutes at 850 x g, at room temperature. Cells from the interface and pellet were collected separately from a cell-trap tube, in accordance with the present invention. Cell fractions were characterized by FACS analysis using FITC- and PE- conjugated anti-CD34 (anti-HPCA-2), anti-CD45 (anti-HLe-1), anti-CD3 (Leu-4) and IgGI antibodies, obtained from Becton Dickinson, Inc. (San Jose, CA). For analysis, cells were labeled with the nuclear dye LDS 751 (Exciton, Inc., Dayton, OH). Statistical analysis was performed on $10^4$ flow events using a FACSCAN system equipped with a LYSYS II program (Becton Dickinson, Inc. (San Jose, CA).

## Example 3

### Density Gradient Separation of PBPC

PBPC cells (025ml), collected according to Example 2 and diluted PBS at a concentration of approximately $5 \times 10^7$ cells/ml) were layered on an OCS particle-based density gradient suspension (15 ml in a 50 ml centrifuge tube). The suspension had a specific density of 1.0605 gr/ml, an osmolality of 280 mOsm/kg $H_2O$ and a pH of 7.4. The layering was performed slowly to avoid mixing of the sample with the solution. A maximum of $2 \times 10^9$ cells was layered per tube. The centrifugation was performed for 30 min. at 850 x g at room temperature. To prevent mixing of the cells and the density gradient solution, the centrifuge was stopped without braking force.

Following centrifugation, the cells were divided into a low-density fraction located at the interface and a high-density fraction forming the pellet. Each cell fraction was collected and transferred into another 50 ml polypropylene centrifuge tube. The cells were washed once and stored in $Ca^{++}$ and $Mg^{++}$ free Dulbecco's phosphate buffered saline (D-PBS) at room temperature until further manipulation. The number and functionality of the hematopoietic progenitor cells (CD34 cells) was determined in both cell fractions by FACS analysis and clonogenic assays (CFU) as detailed in Examples 5 and 6, below.

## Example 4

### Effect on Cells of Mixing with Silanized Colloidal Silica Particles

Assessment of the effect of mixing and subsequent incubation of human peripheral blood in silanized colloidal silica particle-based density gradient solutions was carried out by one or more of the methods described below.

After the respective incubations as detailed below, the cells were collected and washed once with PBS. Their ability to form hematopoietic colonies erythroid (CFU-E, BFU-E), granulocyte/macrophage (CFU-6M) and mixed (CFU-GEMM) colonies according to methods known in the art, and as described in Examples 6, below, was screened at the end of each method. In carrying out experiments, Methods 1, 3, and 5 are control incubation conditions for Methods 2, 4, and 6, respectively.

Method 1: An aliquot containing $10^7$-$10^8$ cells in 5 ml of 1 X D-PBS ($Ca^{++}$, $Mg^{++}$ free) was added to a 15 ml polypropylene centrifuge tube. Initial cell number was noted. The cells were incubated for 30 minutes at room temperature with gentle mixing at 15 minute intervals.

Method 2: An aliquot containing $10^7$-$10^8$ cells in 5 ml of OCS density gradient suspension was added to a 15 ml polypropylene centrifuge tube. Initial cell number was noted. The cells were incubated for 30 minutes at room temperature with gentle mixing at 15 minute intervals.

Method 3: An aliquot containing $10^7$-$10^8$ cells in 5 ml 1 X D-PBS ($Ca^{++}$, $Mg^{++}$ free) was placed in a 15 ml polypropylene centrifuge tube. Initial cell number was noted. The cells were incubated for 30 minutes at room temperature, with gentle mixing at 15 minute intervals. The cell mixture was then centrifuged at 550 x g for 10 minutes. Following centrifugation, the supernatant was discarded and the cell pellet was re-suspended in 5 ml of Iscove's medium supplemented with 10% fetal calf serum. The cell mixture was then incubated for 24 hours at room temperature.

Method 4: An aliquot containing $10^7$-$10^8$ cells in 5 ml OCS density gradient suspension was placed in a 15 ml polypropylene centrifuge tube. Initial cell number was noted. The cells were incubated for 30 minutes at room temperature, with gentle mixing at 15 minute intervals. The cell mixture was then centrifuged at 550 x g for 10 minutes. Following centrifugation, the supernatant was discarded and the cell pellet was re-suspended in 5 ml of Iscove's medium supplemented with 10% fetal calf serum. The cell mixture was then incubated for 24 hours at room temperature.

Method 5: An aliquot containing $10^7$-$10^8$ cells in 5 ml of 1 X D-PBS ($Ca^{++}$, $Mg^{++}$ free) supplemented with 10% fetal calf serum was added to a 15 ml polypropylene centrifuge tube. Initial cell number was noted. The cell mixture was then incubated for 24 hours at room temperature.

Method 6: An aliquot containing $10^7$ to $10^8$ cells in 5 ml of OCS density gradient suspension supplemented with 10% fetal calf serum was added to a 15 ml polypropylene centrifuge tube. Initial cell number was noted. The cell mixture was then incubated for 24 hours at room temperature.

## Example 5

### Staining and Quantification of CD34+ Cells by FACS

CD34$^+$ cell quantity was determined by Fluorescence Activated Cell Sorting (FACS) after the cells were labeled with a nuclear dye and mAbs directed to CD34 and CD45. The percentage of CD34 cells present was determined in the gate of nucleated cells. This approach was chosen to avoid interference of non-nucleated particulate material with the accuracy of CD34 cell analysis in the FACS.

A suspension of $2 \times 10^7$ cells/ml was made, using $Ca^{++}$ and $Mg^{++}$ free D-PBS as diluent. Fifty microliters of this cell suspension containing $1 \times 10^6$ cells was added to each well of a 96-well microtiter plate. Fifty microliters of a 20% heat inactivated rabbit serum/D-PBS solution and 10 microliters of 10 $\mu$g/ml LDS 751 (nuclear staining dye) in D-PBS solution were also added to each well, followed by mixing. The plate was covered with foil and incubated for 30 minutes at room temperature. To each control well, was added 10 microliters IgGI-PE. To each test well, was added 10 microliters Anti-CD34-PE, followed by mixing. The plate was covered with foil and incubated for 15 minutes at 4°C. The plate was then centrifuged at 850 x g for 5 minutes at 4°C. The supernatant was then removed by flicking.

Each cell pellet was re-suspended in 200 $\mu$l of cold (4°C) 1xDPBS ($Ca^{++}$ and $Mg^{++}$ free). The plate was then centrifuged at 850 x g for 5 minutes at 4°C, and the resulting supernatant was removed by rapid flicking of the plate. Each cell pellet was re-suspended in 50 $\mu$l 20% heat inactivated rabbit serum solution. anti-CD45-FITC (10 $\mu$l) was added to each control and test well and mixed. The plate was covered with foil and incubated for 30 minutes at 4°C. One hundred microliters of cold (4°C) 1xD-PBS ($Ca^{++}$ and $Mg^{++}$ free) was then added to all control and test wells, and the plate was centrifuged at 850 x g for 5 minutes at 4°C. The plate was flicked rapidly to remove the supernatants, and each cell pellet was re-suspended in 200 $\mu$l cold (4°C) 1xD-PBS ($Ca^{++}$ and $Mg^{++}$ free). The plate was centrifuged at 850 x g for 5 minutes at 4°C, then flicked rapidly to remove supernatant. Each cell pellet was then re-suspended in 200ml 1% paraformaldehyde (4°C). The plate was then covered with foil and stored at 4°C until FACS analysis of the samples.

FACS analysis was performed on $10^4$ flow events using a FACSStar Plus system equipped with a LYSYS 11 statistical analysis program (Becton Dickinson, Inc.). For purposes of analysis, a gate (Region 1) was placed around the nucleated cells determined by LDS 751 staining in FL3 in order to gate out the red cells, platelets and debris. FL1 and FL2 were displayed as a dot plot using Region 1. A gate (Region 2) was placed around the cell population that stained with both the anti-CD45 and anti-CD34 monoclonal antibodies. For purposes of analysis, the percentage of cells that stain with the anti-CD34 (FL2) and anti-CD45 (FL1) monoclonal antibodies was determined in Region 2. This represents the number of CD34 positive cells as a percentage of the total number of nucleated cells. The total number of CD34 positive cells was determined in the unprocessed sample and in the cell fraction obtained from the interface and the pellet after processing of the PBPC samples in the density gradient solution.

## Example 6

### Colony Forming (CFU) Assay

The functional characteristics of the CD34$^+$ cells in a cell sample were determined by the colony formation (CFU) assay. This assay provides quantitation of the number of committed hematopoietic progenitor cells in the cell solution.

Cells were diluted to a concentration of $10^5$ cells in 2mL commercially prepared semisolid methyl cellulose containing various colony stimulating factors and erythropoietin (MethoCult™ H4433 medium, Terry Fox Laboratories, Vancouver).

After 14 days of culture at 37°C, the erythroid (CFU-E, BFU-E), granulocyte/macrophage (CFU-GM) and mixed (CFU-GEMM) colonies were counted under an inverted microscope (40x), according to standard methods. The total number of CFU present in a cell mixture was defined by summation of the different types of colonies counted.

**Example 7**

**Determination of Total Cell Recovery**

The percent cell recovery was determined by the formula:

$$\% \text{ Recovery} = 100 \quad x \quad \frac{\text{Nr. of cells after manipulation}}{\text{Nr. of cells at start}}$$

The percent CD34 cell recovery was determined by the formula:

$$\% \text{ Recovery} = 100 \quad x \quad \frac{\text{Nr. of CD34}^+ \text{ cells after manipulation}}{\text{Number of CD34 cells at start}}$$

The percent clonogenic cell recovery was determined by the formula:

$$\% \text{ Recovery} = 100 \quad x \quad \frac{\text{Nr. clonogenic cells (CFU) after manipulation}}{\text{Nr. of clonogenic cells (CFU) at start}}$$

**Example 8**

**Effect of Radiation on hematopoietic cell recovery**

The effect of irradiated organosilanized colloidal silica particles on hematopoietic cell integrity was tested, using the protocol described in Example 4 as "Methods 3 and 4." OCS particles, OCS particles supplemented with 0.5% PVP, or PBS supplemented with 0.5% PVP (5 ml total volume) was irradiated in a 50 ml conical centrifuge tube by a dose of gamma irradiation (2.5-3.5 megaRads). In these experiments, the density of the OCS particle suspension used was 1.0605 gr/ml, a density that is lower than that of the cells to be isolated, to facilitate subsequent pelleting and quantification of cells present in the tube.

An aliquot containing $3 \times 10^7$ PBPC cells, isolated as described in Example 1, was added to each tube, and initial cell number was noted. The cells were incubated for 30 minutes at room temperature, with gentle mixing at 15 minute intervals. The cell mixture was then centrifuged at 550 x g for 10 minutes. Following centrifugation, the supernatant was discarded and the cell pellet was re-suspended in 5 ml of Iscove's medium supplemented with 10% fetal calf serum. The cell mixture was then incubated for 24 hours at room temperature, and then tested in the CFU assay described in Example 6. Results of this experiment are shown in FIG. 1.

**Example 9**

**Sterile Centrifugation Kit**

A sterile centrifuge kit was prepared for separating CD34$^+$ hematopoietic progenitor cells from peripheral blood mononuclear cells (PBPC) as follows: an OCS particle-based density particle density gradient suspension was prepared as described in Example 1. A stock solution was prepared by mixing 12 parts of the OCS particle suspension with 1 part of 10 x calcium and magnesium-free phosphate buffered saline (PBS) and concentrated PVP (PVP-10; Sigma) to prepare a medium having a density of $1.0605 \pm 0.0005$ gr/ml, pH 7.4, an osmolality 280 mOsm/Kg $H_2O$, and a concentration of 4% PVP. The density of the OCS particle solution may be adjusted on a densitometer to precisely define its accuracy up to at least the fourth decimal place.

Fifteen milliliters of this suspension was added to a polypropylene 50 ml conical centrifuge tube (Baxter). The tube was sealed with the standard screw cap supplied with the tube. The tube and its contents were then exposed to gamma irradiation 2.5-3.5 megaRads by placing it in a chamber. The resulting sterile centrifuge kit was used in separating CD34$^+$ cells from PBPC, as described in Example 4 herein.

**Example 10**

**Enrichment of CD34+ Cells from Cell Mixtures**

A. Preparation of Density Gradients

A.1. PERCOLL® Density Gradient

"PERCOLL® solution was purchased from Pharmacia Biotech (Uppsala, Sweden) and stored at 4°C according to the recommendation of the vendor. A stock solution was prepared by mixing 12 parts of "PERCOLL" with 1 part of 10 x calcium and magnesium-free phosphate buffered saline (PBS). The pH of the solution was adjusted to 7.4 and the osmolality to 280 mOsm/Kg $H_2O$. For use in separating CD34+ cells in a cell mixture, the stock solution was further diluted with calcium and magnesium-free PBS to a density of 1.0605 $\pm$ 0.0005 gr/ml and used at room temperature. It was crucial to adjust the density of the gradient to an accuracy of within $\pm$ 0.0005 gr/ml of 1.0605 gr/ml in order to ensure reproducibility and accuracy of cell separation. This was done by a high precision digital density meter such as DMA 48 (Anton PAAR USA, Ashland, VA). All procedures were performed under sterile conditions and at room temperature.

A.2. Organosilanized Colloidal Silica (OCS) Density Gradient

An OCS particle suspension is an organosilanized colloidal silica particulate suspension prepared as described by Dorn (U.S. Patent 4,927,749). OCS particles ranging in size from 3-22 nm, and particularly from 13-18 nm, form a stable suspension that acts as a density gradient medium for separating heterogeneous mixtures of cells on the basis of buoyant density. Unless otherwise indicated, the OCS particle suspension was prepared in a physiological saline solution to have a density of 1.0605 gr/ml at an osmolality of 280 mOsm/kg $H_2O$, as defined for the enrichment of CD34+ cells from PBSC samples. The suspension was produced under GMP conditions and is sterilized by gamma irradiation in the presence of at least 0.05% polyvinylpyrrolidone (PVP-10; Sigma Chemical Co., St. Louis, MO). The PVP was added as a dry powder to a suspension of OCS particles pre-adjusted to the approximate final desired specific density. PVP concentrations of at least 0.05% and as high as 10% are preferred for use in the methods, apparatus and kit of the invention.

B. Density Gradient Centrifugation of Blood and Bone Marrow Buffy Coats

Apheresed peripheral blood was applied directly onto the density gradient. However, unless otherwise indicated, complete blood and bone marrow aspirates were processed to a buffy coat (removal of red cells), according to standard methods, before they were applied onto the density gradient.

Apheresed blood samples were layered on a "PERCOLL®" gradient previously adjusted to a density of 1.0605 $\pm$ 0.0005 gr/ml, an osmolality of 280 mOsm/Kg $H_2O$, and a pH of 7.4 in a 50 ml conical cell-trap tube or a commercially available tube. The cell-trap tube contained a constriction in a location so that approximately 15ml of "PERCOLL®" was in the lower compartment and 5ml of "PERCOLL®" was above the constriction. It was critical to completely fill the volume under the constriction with "PERCOLL®" to prevent the formation of air bubbles. Generally, 20ml of apheresed blood samples were layered on top of this gradient. The tube was centrifuged at 850 x g for 30 minutes at room temperature. The cells lodged at the interface of the gradient; *i.e.,* on top of "PERCOLL®," were collected by pouring the entire content of the upper compartment of the tube into another 50 ml tube. The cell pellet in the region below the constriction were prevented from pouring off when the tube was inverted.

In order to compare the cell separation method described in the preceding paragraph with conventional methods of separation, the test samples were also was layered on "FICOLL-HYPAQUE" (Pharmacia). The density of the stock "FICOLL solution was at 1.077 $\pm$ 0.001 gr/ml and the osmolality at 320 mOsm/kg $H_2O$ as published by the vendor.

Bone marrow cells were prepared as described in Example 2.

C. Density Adjusted Cell Sorting (DACS)-

C.1. Preparation of Carrier Particles

Silica beads (1.4 microns) obtained from Bangs Laboratories, Carmel, IN were washed with concentrated HCl for 2 hours at room temperature and vortexed intensely every 15 minutes to break up bead clumps. After washing, the beads were centrifuged at 850 g for 5 minutes. The HCl containing supernatant was decanted and the beads were washed with deionized $H_2O$ with intensive vortexing to break up the clumps.

The beads were incubated at room temperature overnight in concentrated nitric acid with constant stirring using a magnetic stirrer. The beads were then centrifuged at 850 g for 5 minutes and washed 3 times with deionized water, using 50 ml of deionized $H_2O$ at each step. The beads were vortexed intensely in between each wash to avoid bead clumping. To prevent microbacterial contamination, the beads were stored at 0-4 degrees centigrade in deionized $H_2O$ until further use.

To silanize the beads, either 3-aminopropyltriethoxysilane, (3-iodopropyl)trimethoxysilane or [1-9trimethoxysilyl]-2(m-(or p) chloromethyl)phenyl] ethane were used. Forty mls of silane solution (a 10% solution in 95% ethanol/deionized $H_2O$) was added per 4 gr of beads. The bead mixture was rotated end over end for 1 hour at room temperature. The beads were centrifuged at 850 g for 5 minutes and the excess silane was washed off using 95% ethanol/deionized $H_2O$ in a volume of 100 ml. The beads were vortexed intensely in between each wash step to avoid bead clumping. After the washing step, the beads can be dried and stored. Alternatively the beads can be stored in 95% ethanol/deionized $H_2O$ in the cold which prevents clumping of the beads.

The silanized beads were incubated overnight in 2.5% glutaraldehyde at room temperature. The next day, the beads were centrifuged at 850 g for 5 minutes and the free glutaraldehyde was washed off with deionized $H_2O$ in a volume of 100 ml per 5 gr beads. The beads were vortexed intensely in between each wash step to avoid bead clumping.

The antibody was added to the aminopropyl beads in an excess, at least 3 mg/m$^2$ total bead surface and rotated end over end overnight at room temperature. The next day, the beads were centrifuged at 850 g for 5 minutes and the free protein was washed off with 100 ml off deionized $H_2O$. The beads were vortexed intensely in between each wash step to avoid bead clumping. The beards were stored in deionized $H_2O$ containing 0.1 sodium azide in the cold. The final bead suspension should contain 70-90% single beads and 10-30% predominantly duplet and triplet beads.

The binding efficiency of the antibody-conjugated beads (in terms of the percent of beads that are coated) can be determined using flow cytometric analysis and a fluoresceinated antibody directed to the coupled antibody. Alternatively, the antibody may be added to the silanized beads directly without the glutaraldehyde linking.

C.2. Anti-CD45-coated Beads

Apheresed blood product was incubated with 1.4$\mu$ aminopropyl glass beads (Bangs Laboratories Inc., Carmel, IN) that were glutaraldehyde coated with an anti-CD45 antibody (clone ALB-12, Biodesign International, Kennebunk, ME) for 45 minutes at room temperature. The entire blood cell mixture was layered on OCS + 0.5% Pip (1.0605 $\pm$ 0.0005 gr/ml, 280 mOsm/Kg $H_2O$, pH 7.4) in a 50 ml tube.

C.3. Goat Anti-Mouse IgG (GAM-IgG)-coated Beads

Cells from the PBSC sample were counted by trypan blue exclusion, washed and re-suspended in PBS supplemented with 0.1% bovine serum albumin (BSA) at a concentration of $10^7$ cells/ml. Anti-CD3, CD4 and CD8 mAbs were added to give a final concentration of 3-10 $\mu$g/ml. The cells were incubated for 30 minutes on ice and then centrifuged. The cell pellet was re-suspended in 0,1% BSA in PBS at a cell concentration of $10^7$ cells/ml. Goat anti-mouse IgG (GAM-IgG) coated DACS beads were counted and added to the cells to give bead:cell ratios of between 4:1 and 8:1. The beads and calls were incubated for 15 minutes. During the incubation the cell:bead mixture was gently re-suspended every 5 minutes. The cell-bead mixture was then layered onto OCS density gradient suspension and centrifuged at room temperature at 850 x g for 30 minutes. The cells at the interface were harvested by decanting the centrifugation tube, counted and prepared for FACS analysis. To examine non-specific depletion, a similar procedure was used but the incubation with the monoclonal antibodies was omitted.

Experiments to examine the efficacy of DACS for T lymphocyte purging were performed using mobilized PBSC preparations and anti-CD3, CD4 and CD8 mAbs. The yield of T lymphocytes was monitored using an anti-CD2 mAb in order to avoid artifacts associated with mAbs recognizing the same epitopes on the differentiation antigens.

D. Conjugation of Cell Attachment Molecules to Carrier Particles

D.1. Monoclonal Antibodies

Phycoerythrin-conjugated (PE-CD34) anti-CD34 monoclonal antibodies (hematopoietic progenitor cell marker) and fluorescein isothiocyanate-conjugated (FITC-CD45) anti-CD45 monoclonal antibodies (pan-leukocyte marker) were obtained from Becton Dickinson, Inc. (San Jose, CA). Unconjugated antibodies directed to CD45, CD 16 (granulocytes, monocytes), CD3 (T lymphocytes), CD14 (monocytes) were prepared in the laboratory, according to methods well known in the art.

The anti-CD3 (clone Leu4), CD4 and CD8 mAbs were obtained from Dr. E. Engleman of Stanford University Hospital Blood Bank. In addition, FITC-CD2, PE-CD34 and FITC-CD45 were purchased from the same vendor.

Antibodies were conjugated to either goat anti-mouse coated magnetic beads or to goat anti-mouse coated aminopropyl glass beads by overnight incubation at room temperature. Alternatively, the antibodies could be bound directly to these beads without the goat anti-mouse bridge or could be bound via an avidin-biotin coupling reaction. In addition the antibodies could be cleaved into Fab2 fragments in order to reduce non-specific binding of cells to the beads via their Fc portion.

D.2. Goat Anti-mouse IgG (GAM-IgG)

Mouse IgGI conjugated with either FITC ar PE were purchased from Becton Dickinson (San Jose, CA) and used as isotype control.

Several different procedures were used to prepare DACS beads coated with goat anti-mouse IgG (GAM-IgG) (Biodesign International). The goal was to maximize specific depletion and minimize non-specific depletion during DACS. DACS beads were covalently coated either with GAM-IgG or F(ab)$_2$ GAM-IgG (East Coast Biologicals) in a random orientation or GAM-IgG in a specific orientation to maximize the availability of the Ig binding sites to attach to antigen. In addition, some preparations of beads were coated with polyethylene glycol (PEG) (Shearwater) to reduce the non-specific affinity of the beads for protein or cells. The different chemistries are outlined below.

**Random GAM-IgG or F(ab)$_2$ GAM-IgG**

Silica beads were coated with aminopropyltriethoxysilane (Sigma) which has -NH$_2$ groups available to be conjugated to the -CHO of glutaraldehyde (Sigma). According to the chemistry employed, glutaraldehyde then forms a bridge and binds to the -NH$_2$ group on lysines throughout the GAM-IgG or F(ab)$_2$ GAM-IgG molecules, when both antibody and glutaraldehyde were added to the preparation. The orientation of the protein molecule relative to the bead was considered to be random, on the grounds that any lysine with an -NH$_2$ side-chain will potentially conjugate to the -CHO of the glutaraldehyde. This gives an ATES-GLUT-IgG chain with the IgG or IgG-F(ab)$_2$ in a random orientation.

**GAM-IgG in a specific orientation**

Silica beads were coated with aminopropyltriethoxysilane having -NH$_2$ groups available to be conjugated to the -CHO group of the carbohydrate on the glycosylated region of the Fc portion of the GAM-IgG molecule. The GAM-IgG molecule is only glycosylated on the Fc portion so that linking GAM-IgG to the bead surface through this region will have the effect of orienting the IgG molecule with the Fc portion next to the bead surface and the IgG binding sites away from the surface. This treatment theoretically increases the number of binding sites available to bind antigen and decreases the availability of the FC portion to bind Fc receptors. This gives an ATES-CHO-IgG chain with the IgG in a specific orientation.

**PEG-aldehyde random GAM-IgG**

Silica beads were coated with aminopropyltriethoxysilane which has -NH$_2$ groups available to be conjugated to one -CHO group of an aldehyde-PEG-aldehyde molecule where PEG is sandwiched between two aldehyde residues. GAM-IgG then bound to the other -CHO residue to give an ATES-CHO-PEG-CHO-IGg chain with the IgG in a random orientation.

E. Analysis

Antibody staining, FACS analysis and CF assays were carried out as described in Examples 5 and 6.

F. Long-Term Culture Initiating Cell (LTC-IC) Assay/Functional Determination of Uncommitted CD34$^+$ Cells

The number of uncommitted hematopoietic progenitor cells in a cell mixture was determined by the long term culture initiating culture. The cells were seeded on an irradiated stroma feeder layer and a determination of CFU's was made in function of time. Hematopoietic stem cells were able to self-renew and gave rise to CFU's in this system for a period that exceeded 5 weeks. Long term bone marrow stromal cultures were initiated in 96 well plates ($10^6$ cells in 200 $\mu$l per well) in $\alpha$-MEM medium supplemented with 12.5% horse serum, 12.5% fetal calf serum, 2 mM L-glutamine, 0.2 mM i-inositol, 20 $\mu$M folic acid, $10^{-4}$ M 2-mercaptoethanol and were kept at 33°C in a humidified atmosphere. At weekly intervals, half the medium was removed and replaced by an equal volume of fresh medium. After 2 weeks of culture, the confluent stroma layers were gamma irradiated (2000 rad) to kill endogenous hematopoietic cells. Unfractionated samples and cell preparations after separation were seeded onto the irradiated stroma layers in the same medium supplemented with $10^{-6}$M hydrocortisone. After five weeks of culture the adherent and non-adherent cells were collected and screened in the CFU assay as in Part G, above.

G. Natural Killer (NK) Cell Assay

K562 target cells were labeled with 100 $\mu$Ci $^{51}$Cr for 1 hour at 37'C and then washed four times and counted. The target cells were co-cultured for 4 hours in V-bottom 96 well multiwell plates with unfractionated apheresed blood and cells from the different fractions after cell separation. Effector and target cells were mixed at different ratios, 100:1, 50:1, 25:1 and 12:1. For example, the 100:1 ratio contained $5 \times 10^5$ effector cells and $5 \times 10^3$ target cells. After the incubation period, 100$\mu$l of the supernatant was harvested and counted in a scintillation counter. Maximal and spontaneous $^{51}$Cr release was measured counting either 50$\mu$l of the stock target solution and supernatant from the effectors by themselves, respectively. The percent cytotoxicity was determined according to formula:

$$\text{Percent Cytotoxicity} = \frac{\text{cpm experiment - cpm spontaneous release}}{\text{cpm maximal release - cpm spontaneous release}}$$

J. Mixed Lymphocyte Culture and Natural Suppressor Cell Activity

Cells from two different buffy coats were mixed in a flat bottom 96 well multiwell plate at $10^5$ cells of each. One of the buffy coats received 3000 rad and was referred to as the "stimulators". The other buffy coat was used untreated and referred to as "responders." Unfractionated apheresed peripheral blood products (APBL) or cells from the different density fractions were added to these co-cultures at $10^5$ cells per well. These cells were referred to as "suppressors" and received 1500 rad prior to being added to the MLR. The cells were cultured for 5 days and then pulsed with [H]-thymidine (1 $\mu$Ci/well). 18 hours later, the cells were harvested and the amount of thymidine incorporated determined in a scintillation counter. The percent suppression induced by the suppressor cells was determined by the formula:

$$\text{Percent Suppression} = \frac{\text{cpm control - cpm experiment}}{\text{cpm experiment}}$$

## Example 11

## Purging Tumor Cells from Peripheral Blood

The human B-cell lymphoma cell line SU-DHL4 (Stanford University Blood Bank, Stanford, California) was used as a standard experimental model to model tumor cell contamination of peripheral blood. This cell line contains a reciprocal translocation between the long arms of human chromosomes 14 and 18, resulting in a juxtaposition of the bcl-2 proto-oncogene to the joining region of the immunoglobulin (Ig) heavy chain gene. This translocation, which leads to elevated levels of the bcl-2 gene product, is found in 85% of follicular small cleaved cell lymphomas and 25% of diffuse large cell lymphomas. The translocation breakpoint in the bcl-2 gene is located in either the major breakpoint region (MBR) spanning approximately 150 bp in the 3' untranslated region (UTR) of the bcl-2 gene or the 500 bp minor cluster region (MCR) located approximately 20 kb downstream of the bcl-2 gene. Using translocation-specific primer pairs and quantitative PCR by limiting dilution analysis, depletion of SU-DHL4 cells in DACS purging experiments can be monitored.
The human SK-BR3 adenocarcinoma cell line (American Type Culture Collection (ATTC), Rockville, MD) was used as a standard experimental model to model breast cancer cell infiltration of peripheral blood. These cells react with the mouse anti-human Her2/Neu monoclonal antibody, indicating they possess a Her2/Neu antigen. Prior to being spiked into normal buffy coats, the SK-BR3 cells were pre-loaded with calcein AM, a fluorogenic substrate so that they can be enumerated by FACS analysis. Alternatively or in addition breast tumor cells contain estrogen receptors and react with antibodies to such receptors and other receptor binding molecules, such as estrogen and estrogen agonists. For purposes of the present invention, such cells can be captured, as in the tumor cell purging methods described herein, by any molecule that binds with sufficient affinity to such a tumor cell antigen, including, but not limited to antibodies, estrogen- or estrogen agonists. In this context, a sufficient affinity of binding is an affinity approximately equal to or within 2 log binding affinity units of the mouse anti-human Her2/Neu monoclonal antibody (HB 8694, 520C9; ATCC, Rockville, MD)

PC Analysis

High molecular weight genomic DNA was prepared using the QIAamp Blood Kit (Qiagen, Chatsworth, Ca.) and quantitated by optical density at 260nm. PCR reactions were done using a Perkin-Elmer model 9600 thermocycler for 35 cycles. Each amplification cycle consisted of 94°C (15"), 55°C (60"), and72°C (15"), with a 72°C (6 min.) extension after the final cycle. For nested reactions, 1 ml of the first-round reaction was used as template. The following oligonucleotides were used for analysis of samples: (i)5-CAGCCTTGAAACATr-GATGG-3 (MBR) (ii)5-ACCTGAGGAGACGGTGACC-3 (J$_H$ Consensus) (iii)5-ATGCTGT-GGTTGATATTTCG-3 (MBR nested) (iv)5-ACCTGAGGAGACGGTGACC-3 (J$_H$ nested Consensus). Amplification products were visualized by agarose gel electrophoresis.

FACS analysis

SK-BR3 cells were pre-loaded by incubating them in calcein AM and then seeded into normal peripheral blood mononuclear cells at a final frequency of 2%. DACS was performed as described below using the mouse monoclonal antibody to human Her-2/Neu. Aliquots of the experimental samples were analyzed by FACS analysis using the LYSYS II program. For the analysis 100,000 events were collected for each sample.

Density Adjusted Cell Sorting

Peripheral blood progenitor cells (PBPC) and buffy coats were obtained from the Bone Marrow Transplantation Laboratory at the Stanford University School of Medicine, Palo Alto, Ca and from the Stanford University Blood Bank, Palo Alto, CA. Monoclonal antibodies to CD9, CD 10; CD 19 and CD20 were obtained from Caltag Laboratories (So. San Francisco, CA). The anti-CD20 mAb (clone IF5) and the anti-human Her2/Neu mAb (HB 8694, 520C9) and the SK-BR3 adenocarcinoma cell line were obtained from the ATCC (Rockville, MD). Fluorescein isothiocyanate (FITC) conjugated mouse IgG 1 and mouse anti-human CD45 as well as phycoerythrin (PE) conjugated mouse IgG1 and mouse anti-human CD34 antibodies were obtained from Becton Dickinson, Inc (San Jose, Ca.). The FACS procedure and flow cytometric analysis of samples was performed as described above.

Purging and Electrophoretic Analysis of Peripheral Blood Cells

A Peripheral blood buffy coat, spiked (5%) with the t(14;18)+ B cell lymphoma cell line SUDHL-4, was incubated with an anti-B cell mAb cocktail specific for CD9, CD10, CD19 and CD20 cells. After washing, the cell mixture was incubated with GAM-IgG coated DACS beads at a 8:1 bead:cell ratio and then loaded onto OCS suspension at densities ranging from 1.0605 to 1.0720, with comparable results. Following centrifugation for 30 minutes at 850g, DNA was prepared from the cells at the interface and analyzed by standard gel electrophoretic methods at different concentrations (1mg, 0.1mg, 0.01mg, 0.001mg) by two rounds of PCR using t(14;18) specific primers. The number of t(14;18) translocations was furthermore determined by limiting dilution PCR. As control, the same protocol was used on un-spiked cells, SUDHL-4 spiked sample and SUDHL-4 spiked sample treated with DACS beads without prior incubation with the anti-B cell mAbs cocktail.

Centrifugation of the cell mixture over OCS suspension did not result in a reduction of the PCR signal. Whereas the DACS beads alone induced a small reduction of the PCR signal, the PCR signal was completely absent in the SUDHL-4 spiked sample that was treated with both the anti-B cell mAb cocktail and the DACS beads. The determination of the number of t(14;18) translocations by limiting dilution PCR, showed that in the latter experimental group the number of SUDHL-4 cells was reduced by 2-3 logs (100-1000-fold). Preliminary experiments indicate that an additional round of DACS in this context further decreases the number of SUDHL-4 cells by approximately an additional log.

**Example 12**

**Isolation of Dendritic Cells**

Buffy coats prepared from one unit of blood from HLA-A0201 positive volunteer healthy donors are obtained from the Stanford University Blood Center (Stanford, CA). Cells are harvested from the leukopacs, diluted to 60 mL using Ca$^{++}$/Mg$^{++}$ free phosphate buffered saline (D-PBS; Gibco Laboratories, Grand Island, NY) and layered over two 15 mL columns of OCS separation medium (density 1.0720 gr/ml, pH 7.4, 280 mOsm/kg H$_2$O) in 50 mL centrifuge tubes, preferably cell-trap tubes. The tubes are centrifuged at 1000 x g for 35 minutes at room temperature. The centrifuge run is allowed to stop without braking and the peripheral blood mononuclear cells (PBMC), present at the interface, are harvested.

PBMC are re-suspended in D-PBS, centrifuged once at 650 x g for 10 minutes and twice more at 200 x g for 5 minutes to remove platelets. Platelet-depleted PBMC are re-suspended in 60 mL of D-PBS, layered on top of two columns of 15 mL of OCS (density 1.0610 gr/ml, 280 mOsm/kg H$_2$O) in a cell-trap tube of the present invention and centrifuged at 650 x g for 25 minutes at 4°C without braking. The resulting interface (primarily monocytes) and pellet cells (primarily lymphocytes) are harvested and washed with D-PBS by centrifugation at room temperature (once at 650 x g for 10 minutes and twice thereafter at 200 x g for 5 minutes).

In instances where the dendritic cells are used to generate peptide-specific cytotoxic T lymphocytes (CTL) for purposes of elucidating their antigen presentation function, the interface fraction (mostly monocytes) is re-suspended in cold pooled human AB serum (Irvine Scientific, Santa Ana, CA) to which an equal volume of 80% AB serum 20% dimethyl sulfoxide (DMSO) (Sigma Chemical Company, St. Louis, MO) is added dropwise. The resulting cell suspension is aliquoted into cryovials and frozen in liquid nitrogen. The monocytes can be used for re-stimulation of CTL for expansion.

The pellet fraction is re-suspended in 100 mL of AB Culture Medium, inoculated into two T-75 tissue culture flasks and cultured in a humidified 5% $CO_2$ incubator for 40 hours. Following the incubation, the non adherent cells are harvested by moderate pipeting, washed and re-suspended at a concentration of 2 - 5 x $10^6$ cells/mL in AB Culture Medium. The cell suspension is overlayered over four columns of 4.0 mL OCS separation medium (density 1.0565 gr/ml, pH 7.4, 280 mOsm/kg $H_2O$), in AB Culture Medium and centrifuged at 650 x g for 20 minutes at room temperature without braking.

The interface and pellet cells are harvested and washed in AB Culture Medium (Basal RPMI-1640 medium, Gibco Laboratories, Grand Island, NY) by centrifugation once at 650 x g for 10 minutes and twice thereafter at 200 x g for 5 minutes each at room temperature. The yield and viability of both cell fractions is estimated by counting on a hemocytometer using trypan blue exclusion.

The purity of dendritic cells in the interface fraction is quantified following analysis on a flow cytometer (FACS). Dendritic cells are characterized as negative for cell phenotype markers CD3 (T lymphocytes), CD14 (monocytes), CD16 (NK cells) and CD20 (B-cells) and positive for HLA class II expression using dual staining with HLA-DR (on the FTTC channel) and a cocktail of CD3, CD14, CD16, CD20 (on the PE channel). Dual staining with IgG2a on both the FITC and PE channels can be used as isotype control.

The morphology of the cells can also be evaluated using photomicroscopy. The DC enriched fraction contains large sized veiled cells with cytoplasmic processes extending from the cell surface, features characteristic of DC.

**Claims**

1.  A composition for use in cell separation, comprising
    a silanized colloidal silica particle-based cell separation medium containing at least about 0.05% polylactam, wherein said separation medium containing said polylactam is sterilized.

2.  The composition of claim 1, wherein said colloidal silica particle-based cell separation medium is composed of organosilanized silica particles.

3.  The composition of claim 1 or 2, wherein said composition is sterilized by autoclaving or by ionizing radiation.

4.  The composition of claim 3, wherein said ionizing radiation is selected from gamma irradiation and E-beam irradiation.

5.  The composition of any of claims 1 to 4, wherein said polylactam is polyvinylpyrrolidone present at a concentration between about 0.1 and about 10 percent.

6.  The composition of any of claims 1 to 5, wherein said particle is a microsphere having a diameter of between about 0.003 and 50 microns.

7.  The composition of any of claims 1 to 6, for use in isolating a selected rare blood cell from a cell mixture, wherein said silanized colloidal silica particle suspension has a specific density within about $\pm$ 0.0005 gr/ml of said selected cell.

8.  The composition of claim 7, wherein the rare blood cell is selected from the group consisting of hematopoietic progenitor CD34+ cells, dendritic cells, natural killer cells, natural suppressor cells, cytotoxic T cells, tumor cells and nucleated fetal cells.

9.  The composition of claim 8, wherein said rare blood cell is a hematopoietic progenitor CD34+ cell, said cell mixture comprises peripheral blood mononuclear cells, and said organosilanized colloidal silica particle suspension has a specific density of 1.0605 gr/ml.

10. The composition of claim 8, wherein said rare blood cell is a hematopoietic progenitor CD34+ cell, said cell mixture consists of bone marrow cells, and said organosilanized colloidal silica particle suspension has a specific density of 1.0685 gr/ml.

11. The composition of claim 8, wherein said rare blood cell is a dendritic cell, said cell mixture comprises peripheral blood mononuclear cells, and said organosilanized colloidal silica particle suspension has a specific density selected from the group consisting of 1.0770 gr/ml, 1.0720 gr/ml, 1.0650 gr/ml, 1.0610 gr/ml, and 1.0565 gr/ml.

12. A kit for cell separation, comprising
a centrifugable container, and
a silanized colloidal silica particle-based cell separation suspension according to any of claims 1 to 11.

13. A method of sterilizing a silanized silica particle-based cell separation medium, comprising subjecting said separation medium to autoclaving or ionizing radiation in the presence of at least about 0.05 percent polylactam.

14. The method of claim 13, wherein said polylactam is polyvinylpyrrolidone present at a concentration between about 0.1 and about 10 percent.

15. The method of either of claims 13 or 14, wherein said silica particle is an organosilanized colloidal silica particle.

16. A method of isolating a selected rare blood cell from a cell mixture, comprising layering the mixture containing said rare hematopoietic cell on a silanized colloidal silica particle-based cell separation medium according to any of claims 1 to 6, and centrifuging said mixture at a centrifugal force sufficient to pellet cells having a specific density that is greater than the specific density of the selected cell.

17. The method of claim 16 wherein said mixture containing said rare cell and said cell separation medium are contained in a centrifugation tube having side walls and a closed bottom,
a constriction member disposed within the tube, said constriction member positioned and constructed to retain fluid in the bottom portion of the tube below the constriction member, when the tube is inverted, and
wherein said cell-separation medium has a specific density that is within $\pm 0.0005$ gr/ml of the specific density of the selected cell type and said medium is contained in the bottom portion of the tube and extending above said constriction member to a level above an opening formed by said constriction member, such that cells which are captured at an interface between the cell-separation medium and a lower-density medium, after centrifugation, are discharged with the lower-density medium when the tube is inverted.

18. The method of claim 17, wherein said tube includes a closed top, a first port in said top through which fluid material can be introduced into the tube, a second port in said top, and a closed fluid channel communicating the second port with the bottom of the tube below said constriction member.

19. The method of claim 16, wherein the selected rare cell is a functional hematopoietic progenitor cell, the cell mixture is a peripheral blood mononuclear cell mixture, and the specific density of the cell separation density gradient suspension is 1.0605 gr/ml.

20. The method of claim 16, wherein the selected rare cell is a functional hematopoietic progenitor cell, the cell mixture is a bone marrow cell mixture, and the specific density of the cell separation density gradient suspension is 1.0685 gr/ml.

21. The method of claim 16, wherein the selected rare blood cell is a tumor cell, the cell mixture is derived from blood or bone marrow, and wherein the cell separation medium has a specific density selected from the range 1.0490-1.0580 gr/ml.

22. The method of claim 16, wherein the selected rare blood cell is a dendritic cell, and wherein said cell separation medium has a specific density selected from the group consisting of 1.0770 gr/ml, 1.0720 gr/ml, 1.0650 gr/ml, 1.0610 gr/ml, and 1.0565 gr/ml.

23. The method of claim 16, wherein the selected rare blood cell is a cytotoxic T lymphocyte and wherein said cell separation medium has a specific density selected from the group consisting of 1.0720 gr/ml, 1.0610 gr/ml and 1.0565 gr/ml.

24. The method of any of claims 16 to 23, which further includes adding to the cell mixture a suspension which includes a silanized silica particle to which is attached a cell antigen-specific binding molecule.

25. The method of claim 24, for use in depleting T-cell lymphocytes, wherein said cell antigen-specific binding molecule binds to an antigen present on said T-cell lymphocytes.

26. The method of claim 25, wherein said cell antigen-specific binding molecule is selected from the group consisting of anti-CD3, anti-CD4 and anfi-CD8 mouse monoclonal antibodies.

**Patentansprüche**

1. Zusammensetzung zur Verwendung in der Zelltrennung, umfassend ein Zelltrennmedium auf der Basis von silanisierten, kolloidalen Silikapartikeln, enthaltend mindestens etwa 0,05% Polylactam, wobei das Trennmedium, das das Polylactam enthält, sterilisiert ist.

2. Zusammensetzung gemäß Anspruch 1, wobei das Zelltrennmedium auf der Basis von kolloidalen Silikapartikeln aus organosilanisierten Silikapartikeln zusammengesetzt ist.

3. Zusammensetzung gemäß Anspruch 1 oder 2, wobei die Zusammensetzung durch Autoklavieren oder durch ionisierende Strahlung sterilisiert wird.

4. Zusammensetzung gemäß Anspruch 3, wobei die ionisierende Strahlung ausgewählt ist aus Gammabestrahlung und Elektronenbestrahlung.

5. Zusammensetzung gemäß einem der Ansprüche 1 bis 4, wobei das Polylactam Polyvinylpyrrolidon ist, das in einer Konzentration zwischen etwa 0,1 und etwa 10 Prozent vorliegt.

6. Zusammensetzung gemäß einem der Ansprüche 1 bis 5, wobei der Partikel eine Mikrosphäre ist, die einen Durchmesser zwischen etwa 0,003 und 50 Mikrons hat.

7. Zusammensetzung gemäß einem der Ansprüche 1 bis 6, zur Verwendung in der Isolierung einer ausgewählten seltenen Blutzelle aus einem Zellgemisch, wobei die Suspension von silanisierten, kolloidalen Silikapartikeln eine spezifische Dichte innerhalb etwa $\pm 0,0005$ gr/ml der ausgewählten Zelle hat.

8. Zusammensetzung gemäß Anspruch 7, wobei die seltene Blutzelle ausgewählt ist aus der Gruppe bestehend aus hämatopoetischen Vorläufer-CD34[+]-Zellen, dendritischen Zellen, natürlichen Killerzellen, natürlichen Suppressorzellen, cytotoxischen T-Zellen, Tumorzellen und kernhaltigen fetalen Zellen.

9. Zusammensetzung gemäß Anspruch 8, wobei die seltene Blutzelle eine hämatopoetische Vorläufer-CD34[+]-Zelle ist, das Zellgemisch mononukleäre Zellen des peripheren Blutes umfasst und die Suspension von organosilanisierten, kolloidalen Silikapartikeln eine spezifische Dichte von 1,0605 gr/ml hat.

10. Zusammensetzung gemäß Anspruch 8, wobei die seltene Blutzelle eine hämatopoetische Vorläufer-CD34[+]-Zelle ist, das Zellgemisch aus Knochenmarkszellen besteht und die Suspension von organosilanisierten, kolloidalen Silikapartikeln eine spezifische Dichte von 1,0685 gr/ml hat.

11. Zusammensetzung gemäß Anspruch 8, wobei die seltene Blutzelle eine dendritische Zelle ist, das Zellgemisch mononukleäre Zellen des peripheren Blutes umfasst und die Suspension von organosilanisierten, kolloidalen Silikapartikeln eine spezifische Dichte ausgewählt aus der Gruppe bestehend aus 1,0770 gr/ml, 1,0720 gr/ml, 1,0650 gr/ml, 1,0610 gr/ml und 1,0565 gr/ml hat.

12. Kit zur Zelltrennung umfassend einen zentrifugierbaren Behälter und eine Zelltrennsuspension auf der Basis von silanisierten, kolloidalen Silikapartikeln gemäß einem der Ansprüche 1 bis 11.

13. Verfahren zum Sterilisieren eines Zelltrennmediums auf der Basis von silanisierten Silikapartikeln, umfassend das Aussetzen des Trennmediums einer Autoklavierung oder ionisierter Strahlung in Anwesenheit von mindestens etwa 0,05 Prozent Polylactam.

14. Verfahren gemäß Anspruch 13, wobei das Polylactam Polyvinylpyrrolidon ist, das in einer Konzentration zwischen etwa 0,1 und etwa 10 Prozent vorliegt.

15. Verfahren gemäß Anspruch 13 oder 14, wobei der Silikapartikel ein organosilanisierter, kolloidaler Silikapartikel ist.

16. Verfahren zum Isolieren einer ausgewählten seltenen Blutzelle aus einem Zellgemisch, umfassend das Überschichten eines Zelltrennmediums auf der Basis von silanisierten, kolloidalen Silikapartikeln gemäß einem der Ansprüche 1 bis 6 mit dem Gemisch, das die seltenen hämatopoetischen Zellen enthält, und das Zentrifugieren des Gemischs mit einer Zentrifugalkraft, die ausreicht, um Zellen zu pelletieren, die eine spezifische Dichte haben, die größer ist als die spezifische Dichte der ausgewählten Zelle.

17. Verfahren gemäß Anspruch 16, wobei das Gemisch, das die seltenen Zellen enthält, und das Zelltrennmedium in einem Zentrifugenröhrchen enthalten sind, das
Seitenwände und einen geschlossenen Boden hat,
ein Konstriktionselement hat, welches sich in dem Röhrchen befindet, wobei das Konstriktionselement so positioniert und konstruiert ist, dass die Flüssigkeit in dem Bodenteil des Röhrchens unterhalb des Konstriktionselements zurückgehalten wird, wenn das Röhrchen umgedreht wird, und
wobei das Zelltrennmedium eine spezifische Dichte hat, die innerhalb von $\pm 0,0005$ gr/ml der spezifischen Dichte des ausgewählten Zelltyps liegt, und das Medium in dem Bodenteil des Röhrchens enthalten ist und sich über das Konstriktionselement bis zu einem Stand über eine (Öffnung, die von dem Konstriktionselement gebildet wird, ausdehnt, so dass Zellen, die in einer Grenzschicht zwischen dem Zelltrennmedium und einem Medium mit niedrigerer Dichte gefangen sind, nach der Zentrifugation mit dem Medium mit niedrigerer Dichte freigesetzt werden, wenn das Röhrchen umgedreht wird.

18. Verfahren gemäß Anspruch 17, wobei das Röhrchen einen geschlossenen Deckel, eine erste Öffnung in dem Deckel, durch die das flüssige Material in das Röhrchen eingeführt werden kann, eine zweite Öffnung in dem Deckel und einen geschlossenen Flüssigkeitskanal enthält, der die zweite Öffnung mit dem Boden des Röhrchens unterhalb des Konstriktionselements verbindet.

19. Verfahren gemäß Anspruch 16, wobei die ausgewählte seltene Zelle eine funktionelle hämatopoetische Vorläuferzelle ist, das Zellgemisch ein Gemisch von mononukleären Zellen des peripheren Blutes ist und die spezifische Zelldichte der Zelltrenndichtegradientensuspension 1,0605 gr/ml ist.

20. Verfahren gemäß Anspruch 16, wobei die ausgewählte seltene Zelle eine funktionelle hämatopoetische Vorläuferzelle ist, das Zellgemisch ein Gemisch von Knochenmarkszellen ist, und die spezifische Dichte der Zelltrenndichtegradientensuspension 1,0685 gr/ml ist.

21. Verfahren gemäß Anspruch 16, wobei die ausgewählte seltene Blutzelle eine Tumorzelle ist, das Zellgemisch von Blut oder Knochenmark abgeleitet ist, und wobei das Zelltrennmedium eine spezifische Dichte ausgewählt aus dem Bereich 1,0490-1,0580 gr/ml hat.

22. Verfahren gemäß Anspruch 16, wobei die ausgewählte seltene Blutzelle eine dendritische Zelle ist, und wobei das Zelltrennmedium eine spezifische Dichte ausgewählt aus der Gruppe bestehend aus 1,0770 gr/ml, 1,0720 gr/ml, 1,0650 gr/ml, 1,0610 gr/ml und 1,0565 gr/ml hat.

23. Verfahren gemäß Anspruch 16, wobei die ausgewählte seltene Blutzelle ein cytotoxischer T-Lymphocyt ist und wobei das Zelltrennmedium eine spezifische Dichte ausgewählt aus der Gruppe bestehend aus 1,0720 gr/ml, 1,0610 gr/ml und 1,0565 gr/ml hat.

24. Verfahren gemäß einem der Ansprüche 16 bis 23, welches außerdem das Hinzufügen einer Suspension zu dem Zellgemisch umfasst, wobei die Suspension einen silanisierten Silikapartikel umfasst, an den ein Zellantigenspezifisches Bindemolekül gebunden ist.

25. Verfahren gemäß Anspruch 24, zur Verwendung zum Entfernen von T-Zell-Lymphocyten, wobei das Zellantigenspezifische Bindemolekül an ein Antigen bindet, das auf den T-Zell-Lymphocyten vorhanden ist.

26. Verfahren gemäß Anspruch 25, wobei das Zellantigen-spezifische Bindemolekül ausgewählt ist aus der Gruppe bestehend aus monoclonalen Anti-CD3-, Anti-CD4- und Anti-CD8-Mausantikörpern.

**Revendications**

1. Composition pour une utilisation dans la séparation cellulaire, comprenant un milieu de séparation cellulaire à base de particules de silice colloïdale silanée contenant au moins environ 0,05 % de polylactame, où ledit milieu de séparation contenant ledit polylactame est stérilisé.

2. Composition selon la revendication 1, dans laquelle ledit milieu de séparation cellulaire à base de particules de silice colloïdale est composé de particules de silice organosilanée.

3. Composition selon la revendication 1 ou 2, où ladite composition est stérilisée par autoclavage ou par des radiations ionisantes.

4. Composition selon la revendication 3, où lesdites radiations ionisantes sont choisies parmi des rayons gamma et un rayonnement par faisceau d'électrons.

5. Composition selon l'une quelconque des revendications 1 à 4, dans laquelle ledit polylactame est de la polyvinylpyrrolidone présente à une concentration située entre environ 0,1 et environ 10 pour cent.

6. Composition selon l'une quelconque des revendications 1 à 5, dans laquelle ladite particule est une microsphère ayant un diamètre entre environ 0,003 et 50 microns.

7. Composition selon l'une quelconque des revendications 1 à 6, pour une utilisation dans l'isolement d'une cellule sanguine rare choisie à partir d'un mélange cellulaire, dans laquelle ladite suspension de particules de silice colloïdale silanée a une densité spécifique à environ $\pm 0,0005$ gr/ml de ladite cellule choisie.

8. Composition selon la revendication 7, dans laquelle la cellule sanguine rare est choisie dans le groupe consistant en des cellules progéniteurs hématopoïétiques CD34$^+$, des cellules dendritiques, des cellules tueuses naturelles, des cellules suppresseurs naturelles, des cellules T cytotoxiques, des cellules tumorales et des cellules foetales nucléées.

9. Composition selon la revendication 8, dans laquelle ladite cellule sanguine rare est une cellule progéniteur hématopoïétique CD34$^+$, ledit mélange cellulaire comprend des cellules mononucléées du sang périphérique, et ladite suspension de particules de silice colloïdale organosilanée a une densité spécifique de 1,0605 gr/ml.

10. Composition selon la revendication 8, dans laquelle ladite cellule sanguine rare est une cellule progéniteur hématopoïétique CD34$^+$, ledit mélange cellulaire consiste en des cellules de la moelle osseuse, et ladite suspension de particules de silice colloïdale organosilanée a une densité spécifique de 1,0685 gr/ml.

11. Composition selon la revendication 8, dans laquelle ladite cellule sanguine rare est une cellule dendritique, ledit mélange cellulaire comprend des cellules mononucléées du sang périphérique, et ladite suspension de particules de silice colloïdale organosilanée a une densité spécifique choisie dans le groupe consistant en 1,0770 gr/ml, 1,0720 gr/ml, 1,0650 gr/ml, 1,0610 gr/ml et 1,0565 gr/ml.

12. Kit de séparation cellulaire, comprenant
un récipient pouvant être centrifugé, et
une suspension de séparation cellulaire à base de particules de silice colloïdale silanée selon l'une quelconque des revendications 1 à 11.

13. Procédé de stérilisation d'un milieu de séparation cellulaire à base de particules de silice silanée, comprenant la soumission dudit milieu de séparation à un autoclavage ou à des radiations ionisantes en présence d'au moins environ 0,05 pour cent de polylactame.

14. Procédé selon la revendication 13, dans lequel ledit polylactame est de la polyvinylpyrrolidone présente à une concentration située entre environ 0,1 et environ 10 pour cent.

15. Procédé selon l'une ou l'autre des revendications 13 ou 14, dans lequel ladite particule de silice est une particule de silice colloïdale organosilanée.

**16.** Procédé d'isolement d'une cellule sanguine rare choisie à partir d'un mélange cellulaire, comprenant le dépôt en couche du mélange contenant ladite cellule hématopoïétique rare sur un milieu de séparation cellulaire à base de particules de silice colloïdale silanée selon l'une quelconque des revendications 1 à 6, et la centrifugation dudit mélange à une force centrifuge suffisante pour culotter les cellules ayant une densité spécifique qui est supérieure à la densité spécifique de la cellule choisie.

**17.** Procédé selon la revendication 16, dans lequel ledit mélange contenant ladite cellule rare et ledit milieu de séparation cellulaire est contenu dans un tube de centrifugation ayant des parois latérales et un fond fermé, un élément de constriction disposé au sein du tube, ledit élément de constriction positionné et construit pour retenir le liquide dans la partie basse du tube au-dessous de l'élément de constriction, lorsque le tube est retourné, et dans lequel ledit milieu de séparation cellulaire a une densité spécifique à ±0,0005 gr/ml de la densité spécifique du type cellulaire choisi et ledit milieu est contenu dans la partie basse du tube et s'étend au-dessus dudit élément de constriction jusqu'à un niveau au-dessus d'une ouverture formée par ledit élément de constriction, de telle manière que les cellules qui sont capturées au niveau d'une interface entre le milieu de séparation cellulaire et un milieu de densité inférieure, après centrifugation, soient déchargées avec le milieu de densité inférieure lorsque le tube est retourné.

**18.** Procédé selon la revendication 17, dans lequel ledit tube comprend une partie haute fermée, un premier pore dans ladite partie haute à travers lequel le matériau liquide peut être introduit dans le tube, un second pore dans ladite partie haute, et un canal à liquide fermé faisant communiquer le second pore avec la partie basse du tube au-dessous dudit élément de constriction.

**19.** Procédé selon la revendication 16, dans lequel la cellule rare choisie est une cellule progéniteur hématopoïétique fonctionnelle, le mélange cellulaire est un mélange de cellules mononucléées du sang périphérique, et la densité spécifique de la suspension à gradient de densité de séparation cellulaire est de 1,0605 gr/ml.

**20.** Procédé selon la revendication 16, dans lequel la cellule rare choisie est une cellule progéniteur hématopoïétique fonctionnelle, le mélange cellulaire est un mélange cellulaire de la moelle osseuse, et la densité spécifique de la suspension à gradient de densité de séparation cellulaire est de 1,0685 gr/ml.

**21.** Procédé selon la revendication 16, dans lequel la cellule sanguine rare choisie est une cellule tumorale, le mélange cellulaire est dérivé du sang ou de la moelle osseuse, et dans lequel le milieu de séparation cellulaire a une densité spécifique choisie dans la plage de 1,0490 à 1,0580 gr/ml.

**22.** Procédé selon la revendication 16, dans lequel la cellule sanguine rare choisie est une cellule dendritique, et dans lequel ledit milieu de séparation cellulaire a une densité spécifique choisie dans le groupe consistant en 1,0770 gr/ml, 1,0720 gr/ml, 1,0650 gr/ml, 1,0610 gr/ml et 1,0565 gr/ml.

**23.** Procédé selon la revendication 16, dans lequel la cellule sanguine rare choisie est un lymphocyte T cytotoxique et dans lequel ledit milieu de séparation cellulaire a une densité spécifique choisie dans le groupe consistant en 1,0720 gr/ml, 1,0610 gr/ml et 1,0565 gr/ml.

**24.** Procédé selon l'une quelconque des revendications 16 à 23, qui comprend, en outre, l'addition au mélange cellulaire d'une suspension qui comprend une particule de silice silanée à laquelle est fixée une molécule de liaison spécifique d'un antigène cellulaire.

**25.** Procédé selon la revendication 24, pour une utilisation dans l'appauvrissement des lymphocytes T, dans lequel ladite molécule de liaison spécifique d'un antigène cellulaire se lie à un antigène présent sur lesdits lymphocytes T.

**26.** Procédé selon la revendication 25, dans lequel ladite molécule de liaison spécifique d'un antigène cellulaire est choisie dans le groupe consistant en des anticorps monoclonaux de souris anti-CD3, anti-CD4 et anti-CD8.

Fig. 1

Fig. 2

**Fig. 3**

**Fig. 4**

Fig. 5

Fig. 6

Fig. 7

Fig. 8

Fig. 9

# Fig. 10

# Fig. 11

Fig. 12

Fig. 13

Fig. 14

Fig. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4927749 A
- WO 9607097 A
- US 9511169 W
- US 9511162 W
- WO 9606679 A

### Non-patent literature cited in the description

- **PERTOFT et al.** *Exp. Cell Res.,* 1966, vol. 46, 621-623
- **PERTOFT et al.** *Exp. Cell Res.,* 1968, vol. 50, 355-368
- **WOLFF et al.** *J. Cell Biol.,* 1972, vol. 55, 579-585
- **PERTOFT et al.** *Exp. Cell Res.,* 1977, vol. 110, 449-457
- **PERTOFT et al.** *Anal. Biochem.,* 1978, vol. 88, 271-282